# EUROPEAN PATENT APPLICATION

(11) **EP 1 304 121 A1**
(43) Date of publication of application: **23.04.2003**
(21) Application number: 00974859.1
(22) Date of filing: 09.11.2000
(51) Int. Cl.: A61K 45/00, C07D 263/32, A61K 31/421, A61K 31/195, A61K 31/235, A61P 3/10, A61P 3/04

(54) **BODY WEIGHT GAIN INHIBITORS**

(30) Priority: 10.11.1999 JP 32031999
(71) Applicant: Takeda Chemical Industries, Ltd., Osaka-shi, Osaka 541-0045 (JP)
(72) Inventor: SUGIYAMA, Yasuo, Kawanishi-shi, Hyogo 666-0111 (JP); ODAKA, Hiroyuki, Kobe-shi, Hyogo 651-1223 (JP); KIMURA, Hiroyuki, Sakai-shi, Osaka 590-0975 (JP)
(74) Representative: Wright, Robert Gordon McRae
(86) International application number: JP0007879
(87) International publication number: WO01034200

(57) **Abstract**

An agent for inhibiting body weight gain derived from a PPARγ agonist-like substance, which contains a PPARδ agonist-like substance, is useful for the treatment of diabetes and the like.

SEQUENCE LISTING
<110> Takeda Chemical Industries, Ltd.
<120> Body Weight Increase Inhibitor
<130> 2668WOOP
<150> JP 11-320319
<151> 1999-11-10
<160> 10
<210> 1
<211> 34
<212> DNA
<213> Artificial Sequence
<220>
<223>
<400> 1
AACGGTACCT CAGCCATGGAG CAGCCTCAG GAGG 34
<210> 2
<211> 34
<212> DNA
<213> Artificial Sequence
<220>
<223>
<400> 2
TAAGTCGACC CGTTAGTACA TGTCCTTGTA GATC 34
<210> 3
<211> 33
<212> DNA
<213> Artificial Sequence
<220>
<223>

<400> 3
TTAGAATTCG ACATGGACAC CAAACATTTC CTG 33
<210> 4
<211> 33
<212> DNA
<213> Artificial Sequence
<220>
<223>
<400> 4
CCCCTCGAGC TAAGTCATTT GGTGCGGCGC CTC 33
<210> 5
<211> 36
<212> DNA
<213> Artificial Sequence
<220>
<223>
<400> 5
TCGACAGGGG ACCAGGACAA AGGTCACGTT CGGGAG 36
<210> 6
<211> 36
<212> DNA
<213> Artificial Sequence
<220>
<223>
<400> 6
TCGACTCCCG AACGTGACCT TTGTCCTGGT CCCCTG 36
<210> 7
<211> 28
<212> DNA
<213> Artificial Sequence

<220>

<223>

<400> 7

CCCAGATCTC CCCAGCGTCT TGTCATTG 28

<210> 8

<211> 28

<212> DNA

<213> Artificial Sequence

<220>

<223>

<400> 8

TCACCATGGT CAAGCTTTTA AGCGGGTC 28

<210> 9

<211> 33

<212> DNA

<213> Artificial Sequence

<220>

<223>

<400> 9

GTGGGTACCG AAATGACCAT GGTTGACACA GAG 33

<210> 10

<211> 33

<212> DNA

<213> Artificial Sequence

<220>

<223>

<400> 10

GGGGTCGACC AGGACTCTCT GCTAGTACAA GTC 33

## Description

### Technical Field

The present invention relates to an agent for inhibiting body weight gain derived from a PPARγ agonist-like substance, which agent is useful for treating diabetes and the like.

### Background Art

As a result of various studies relating to diabetes in recent years, the relationship between retinoid-related receptors and blood glucose, blood lipid and the like has been elucidated.

Particularly, peroxisome proliferator-activated receptor gamma (sometimes to be abbreviated as PPARγ in the present specification), which is one of the retinoid-related receptor ligands and a member of a nucleus hormone receptor super family represented by steroid hormone receptors and thyroid hormone receptors, shows an induced expression at the beginning of differentiation of adipocytes and plays an important role as a master regulator in the differentiation of adipocytes. PPARγ binds to a ligand to form a dimer with retinoid X receptor (RXR) and the dimer binds to a responsive element of a target gene in the nucleus to directly control (activate) the transcription efficiency. In recent years, moreover, it has been clarified that 15-deoxy-Δ^{12,14} prostaglandin J₂, which is a metabolite of prostaglandin D₂, is an endogenous ligand of PPARγ, and that certain insulin sensitizers represented by thiazolidinedione derivatives have a PPARγ ligand activity and the strength of the activity parallels with a hypoglycemic action or adipocyte differentiation promoting action [Cell, vol. 83, p. 803 (1995); The Journal of Biological Chemistry, vol. 270, p. 12953, (1995); Journal of Medicinal Chemistry, vol. 39, p. 655 (1996)]. More recently, it has been elucidated that 1) PPARγ is expressed in the cultured cell derived from human liposarcoma and the addition of PPARγ ligand stops its growth [Proceedings of The National Academy of Sciences of The United States of America, vol. 94, p. 237 (1997)], 2) nonsteroidal anti-inflammatory drugs represented by indomethacin and phenoprofen have a PPARγ ligand activity [The Journal of Biological Chemistry, vol. 272, p. 3406 (1997)], 3) PPARγ is highly expressed in activated macrophage, and the addition of its ligand leads to the inhibition of the transcription of the gene involved in inflammation [Nature, vol. 391, p. 79 (1998)], 4) PPARγ ligand inhibits production of inflammatory cytokines (TNFα, IL-1β, IL-6) by monocyte [Nature, vol. 391, p. 82 (1998)] and the like.

As such PPARγ agonists, phenylalkanoic acid derivatives having substituted hydroxyl groups at the 4-position have been reported (e.g., WO97/31907 WO97/25042).

On the other hand, peroxisome proliferator-activated receptor delta (sometimes to be abbreviated as PPARδ in the present specification) is one kind of the same retinoid-related receptors, and one of the subtypes of peroxisome proliferator-activated receptors (PPAR) like PPARγ. It does not show tissue specificity in the expression site, and is expressed generally. The physiological roles of PPARδ have been scarcely elucidated as yet.

WO97/28149 describes an agonist of such PPARδ. This publication reports that this compound acts as a PPARδ agonist and increases HDL amount in plasma, is effective for the treatment and prophylaxis of coronary atherosclerosis, is effective for the treatment and prophylaxis of coronary atherosclerosis when combined with HMG-CoA reductase inhibitors and the like.

WO99/04815 describes compounds having an agonist activity of both the above-mentioned PPARγ and PPARδ. These compounds have a serum cholesterol lowering effect and the publication describes pharmaceutical compositions thereof.

The agonists of PPARδ and PPARγ have been reported to modulate (up-regulate) the expression of the gene of UCP-2 protein involved in energy balance, body weight control and calory control in preadipocytes, in which PPARδ is expressed highly, and in adipocytes, in which both PPARδ and PPARγ2 are expressed highly [Biochemical and Biophysical Research Communications, 238, 606-611 (1997)].

In addition, it is known that insulin sensitizers (e.g., troglitazone, pioglitazone, rosiglitazone etc.), known to be a highly excellent therapeutic agent of diabetes, have a PPARγ agonist activity [e.g., Journal of Pharmacology and Experimental Therapeutics, 284, 751-759 (1998)]. While such pharmaceutical agent is effective for the treatment of diabetes, a report has documented on a body weight increasing activity of, for example, troglitazone in type 2 diabetes patients (Diabetes, 47, suppl. 1, A18, No. 69, 1998), wherein the administration thereof led to a phenomenon of body weight gain in the patients during treatment. Such body weight gain is among the actions desirably avoided as far as the diabetic patients can, because obesity induces exacerbation of diabetes.

It is therefore an object of the present invention to develop a pharmaceutical agent for the treatment of diabetes and other diseases, which is free of body weight gain of patients, even when a substance having a PPARγ agonist activity effective for the treatment is administered.

### Disclosure of the Invention

The present inventors have first found that the body weight gain of diabetes patients can be suppressed by administering a substance having a PPARδ agonist action during the administration of a substance having a PPARγ agonist action, which resulted in the completion of the present invention.

Accordingly, the present invention relates to
(1) an agent for inhibiting a body weight gain derived from a PPARγ agonist-like substance, which contains a PPARδ agonist-like substance;
(2) the agent according to (1) above, wherein the PPARδ agonist-like substance and the PPARγ agonist-like substance are the same substance;
(3) the agent according to (1) above, wherein the same substance is a compound represented by the formula 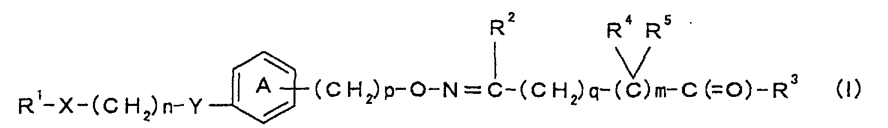 wherein
   - R¹: is an optionally substituted hydrocarbon group or an optionally substituted heterocyclic group;
   - X: is a bond, a group of the formula: -CO-, -CH(OH)- or -NR⁶- (R⁶ is a hydrogen atom or an optionally substituted alkyl group);
   - n: is an integer of 1 to 3;
   - Y: is an oxygen atom, a sulfur atom, a group represented by -SO-, -SO₂- or -NR⁷- (R⁷ is a hydrogen atom or an optionally substituted alkyl group);
   - ring A: is a benzene ring optionally having 1 to 3 additional substituent(s);
   - p: is an integer of 1 to 8;
   - R²: is a hydrogen atom, an optionally substituted hydrocarbon group or an optionally substituted heterocyclic group;
   - q: is an integer of 0 to 6;
   - m: is 0 or 1;
   - R³: is a hydroxy group, -OR⁸ (R⁸ is an optionally substituted hydrocarbon group) or -NR⁹R¹⁰ (R⁹ and R¹⁰ are the same or different and each is a hydrogen atom, an optionally substituted hydrocarbon group, an optionally substituted heterocyclic group, or an optionally substituted acyl group and R⁹ and R¹⁰ are optionally bonded to form a ring); and
   - R⁴ and R⁵: are the same or different and each is a hydrogen atom, an optionally substituted hydrocarbon group, and R⁴ and R² are optionally bonded to form a ring,
   or a salt thereof;
(4) the agent according to (1) above, wherein the same substance is E-4-[4-(5-methyl-2-phenyl-4-oxazolylmethoxy)-benzyloxyimino]-4-phenylbutyric acid;
(5) the agent according to (2) above, which is for the treatment of diabetes;
(6) the agent according to (2) above, which is for the treatment of diabetic complications;
(7) a method for inhibiting a body weight gain derived from a PPARγ agonist-like substance, which comprises administering an effective amount of a PPARδ agonist-like substance to a mammal;
(8) use of a PPARδ agonist-like substance for the production of an inhibitor of a body weight gain derived from a PPARγ agonist-like substance;
(9) a therapeutic agent of diabetes comprising a PPARδ agonist-like substance and a PPARγ agonist-like substance, which inhibits a body weight gain derived from the PPARγ agonist-like substance to not more than about 80%; and
(10) the agent according to (9) above, wherein the PPARδ agonist-like substance and the PPARγ agonist-like substance are the same substance.

The PPARδ agonist-like substance to be used in the present invention needs only to be an agonist of PPARδ. The PPARδ agonist-like substance may be any substance as long as it expresses a PPARδ-related action by regulating the gene expression of a UCP-2 protein relating to biological energy balance, body weight control and calory control and the like, even if it is not a direct agonist of PPARδ.

The PPARδ agonist-like substance is, for example, a substance that clearly shows its action in vitro at a concentration of not more than 10 µM, which is preferably specifically a substance that shows an EC₅₀ of not more than 10 µM in Experimental Example 1 below, and the like.

Preferable examples of the PPARδ agonist-like substance include carbaprostacyclin (cPGI), L-165041 [Journal Biological Chemistry, 274, 6718-6728 (1999, Merck)] and the like. Besides these, the substances described in GB-2,292,885-A activate PPARδ and can be used as a PPARδ agonist-like substance in the present invention. Of these, cPGI is known as an agonist of both PPARδ and PPARα. A substance that can act as an agonist of both PPARδ and PPARγ to be mentioned below is also a PPARδ agonist-like substance that can be used in the present invention.

The PPARγ agonist-like substance to be used in the present invention needs only to be an agonist of PPARγ, and may be any substance as long as it expresses this action.

The PPARγ agonist-like substance is, for example, a substance that clearly shows its action in vitro at a concentration of not more than 10 µM, which is preferably specifically a substance that shows an EC₅₀ of not more than 10 µM in Experimental Example 3 below, and the like.

Preferable examples of the PPARγ agonist-like substance include insulin sensitizers such as troglitazone, rosiglitazone, englitazone, ciglitazone, pioglitazone, PGJ₂, GI-262570, JTT-501, MCC-555, YM-440, KRP-297, CS-011, FK-614 and the like. It is needless to say that a substance that acts as an agonist of both PPARδ and PPARγ to be mentioned below can be used as a PPARγ agonist-like substance in the present invention.

In the present invention, the PPARδ agonist-like substance and the PPARγ agonist-like substance may be the same or different. Examples of such same substance include those known as agonists of both PPARδ and PPARγ, such as YM-16638 (JP-B-63-35626), p-[3-(4-acetyl-3-hydroxy-2-propylphenoxy)propoxy]phenylbutyric acid (CZ 281,130 and WO99/04815), L-165461, L-783483, L-796449 [Journal Biological Chemistry, 274, 6718-6728 (1999, Merck)] and the like.

Examples of the aforementioned same substance include compounds represented by the formula (I) 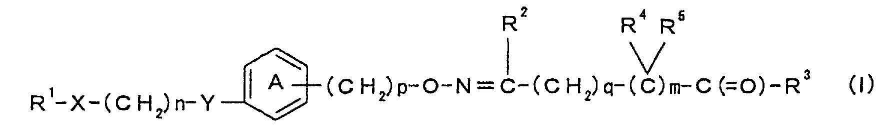 wherein
- R¹: is an optionally substituted hydrocarbon group or an optionally substituted heterocyclic group;
- X: is a bond, a group of the formula: -CO-, -CH(OH)- or -NR⁶- (R⁶ is a hydrogen atom or an optionally substituted alkyl group);
- n: is an integer of 1 to 3;
- Y: is an oxygen atom, a sulfur atom, a group represented by -SO-, -SO₂- or -NR⁷- (R⁷ is a hydrogen atom or an optionally substituted alkyl group);
- ring A: is a benzene ring optionally having 1 to 3 additional substituent (s) ;
- p: is an integer of 1 to 8;
- R²: is a hydrogen atom, an optionally substituted hydrocarbon group or an optionally substituted heterocyclic group;
- q: is an integer of 0 to 6;
- m: is 0 or 1;
- R³: is a hydroxy group, -OR⁸ (R⁸ is an optionally substituted hydrocarbon group) or -NR⁹R¹⁰ (R⁹ and R¹⁰ are the same or different and each is a hydrogen atom, an optionally substituted hydrocarbon group, an optionally substituted heterocyclic group, or an optionally substituted acyl group and R⁹ and R¹⁰ are optionally bonded to form a ring); and
- R⁴ and R⁵: are the same or different and each is a hydrogen atom, an optionally substituted hydrocarbon group, and R⁴ and R² are optionally bonded to form a ring, or a salt thereof; the compounds (e.g., 5-[3-[4-[(5-methyl-2-phenyl-1,3-thiazol-4-yl)methoxy]phenyl]propyl]-1,3-oxazolidine-2,4-dione and the like) described in EP-A612743, EP-A629624 and the like, and the like.

The formula (I) is described in detail in the following.

### (1) Definition of R¹

In the formula (I), examples of the hydrocarbon group of the "optionally substituted hydrocarbon group" represented by R¹ include aliphatic hydrocarbon group, alicyclic hydrocarbon group, alicyclic-aliphatic hydrocarbon group, aromatic-aliphatic hydrocarbon group and aromatic hydrocarbon group. These hydrocarbon groups preferably have 1 to 14 carbon atoms.

### (1-1) Definition of hydrocarbon group of R¹

The aliphatic hydrocarbon group is preferably aliphatic hydrocarbon group having 1 to 8 carbon atoms. Examples of the aliphatic hydrocarbon group include saturated aliphatic hydrocarbon group having 1 to 8 carbon atoms (e.g., alkyl group etc.), such as methyl, ethyl, propyl, isopropyl, butyl, isobutyl, sec.-butyl, t.-butyl, pentyl, isopentyl, neopentyl, hexyl, isohexyl, heptyl, octyl and the like; and unsaturated aliphatic hydrocarbon group having 2 to 8 carbon atoms (e.g., alkenyl group having 2 to 8 carbon atoms, alkadienyl group having 4 to 8 carbon atoms, alkenylalkynyl group having 2 to 8 carbon atoms, alkadiinyl group having 4 to 8 carbon atoms etc.), such as ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, ethynyl, 1-propinyl, 2-propinyl, 1-butynyl, 2-butynyl, 3-butynyl, 1-pentinyl, 2-pentinyl, 3-pentinyl, 4-pentinyl, 1-hexynyl, 3-hexynyl, 2,4-hexadiinyl, 5-hexynyl, 1-heptinyl, 1-octynyl and the like.

The alicyclic hydrocarbon group is preferably alicyclic hydrocarbon group having 3 to 7 carbon atoms. Examples of the alicyclic hydrocarbon group include saturated alicyclic hydrocarbon group having 3 to 7 carbon atoms, (e.g., cycloalkyl group etc.), such as cyclopropyl, cyclobutyl, cyclopentyl, cyclohexyl, cycloheptyl and the like; unsaturated alicyclic hydrocarbon group having 5 to 7 carbon atoms (e.g., cycloalkenyl group, cycloalkadienyl group etc.), such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl and the like.

Examples of the alicyclic-aliphatic hydrocarbon group include one wherein the above-mentioned alicyclic hydrocarbon group and the aliphatic hydrocarbon group are bonded (e.g., cycloalkyl-alkyl group, cycloalkenyl-alkyl group etc.). Of these, an alicyclic-aliphatic hydrocarbon group having 4 to 9 carbon atoms is preferable. Examples of the alicyclic-aliphatic hydrocarbon group include cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like.

The aromatic-aliphatic hydrocarbon group is preferably aromatic-aliphatic hydrocarbon group having 7 to 13 carbon atoms (e.g., aralkyl group having 7 to 13 carbon atoms, arylalkenyl group having 8 to 13 carbon atoms etc.). Examples of the aromatic-aliphatic hydrocarbon group include phenylalkyl having 7 to 9 carbon atoms such as benzyl, phenethyl, 1-phenylethyl, 1-phenylpropyl, 2-phenylpropyl, 3-phenylpropyl and the like; naphthylalkyl having 11 to 13 carbon atoms such as α-naphthylmethyl, α-naphthylethyl, β-naphthylmethyl, β-naphthylethyl and the like; phenylalkenyl having 8 to 10 carbon atoms such as styryl and the like; naphthylalkenyl having 12 to 13 carbon atoms such as 2-(2-naphthylvinyl) and the like, and the like.

The aromatic hydrocarbon group is preferably aromatic hydrocarbon group having 6 to 14 carbon atoms (e.g., aryl group etc.). Examples of the aromatic hydrocarbon group include phenyl, naphthyl, anthryl, phenanthryl, acenaphtylenyl, biphenylyl and the like, particularly preferably phenyl, 1-naphthyl, 2-naphthyl and the like.

### (1-2) Definition of heterocyclic group of R¹

In the formula (I), examples of the heterocyclic group of the "optionally substituted heterocyclic group" represented by R¹ include a 5 to 7-membered monocyclic heterocyclic group or a fused heterocyclic group, containing, besides carbon atom, 1 to 4 heteroatoms selected from oxygen atom, sulfur atom and nitrogen atom, as a ring-constituting atom. Examples of the fused heterocycle include fused rings of the 5 to 7-membered monocyclic heterocycle with a 6-membered ring containing 1 or 2 nitrogen atoms, a benzene ring or a 5-membered ring containing one sulfur atom.

Examples of the heterocyclic group include aromatic heterocyclic groups such as 2-pyridyl, 3-pyridyl, 4-pyridyl, 2-pyrimidinyl, 4-pyrimidinyl, 5-pyrimidinyl, 6-pyrimidinyl, 3-pyridazinyl, 4-pyridazinyl, 2-pyrazinyl, 1-pyrrolyl, 2-pyrrolyl, 3-pyrrolyl, 1-imidazolyl, 2-imidazolyl, 4-imidazolyl, 5-imidazolyl, 1-pyrazolyl, 3-pyrazolyl, 4-pyrazolyl, isoxazolyl, isothiazolyl, 2-thiazolyl, 4-thiazolyl, 5-thiazolyl, 2-oxazolyl, 4-oxazolyl, 5-oxazolyl, 1,2,4.-oxadiazol-5-yl, 1,3,4-oxadiazol-2-yl, 1,3,4-thiadiazol-2-yl, 1,2,4-triazol-1-yl, 1,2,4-triazol-3-yl, 1,2,3-triazol-1-yl, 1,2,3-triazol-2-yl, 1,2,3-triazol-4-yl, tetrazol-1-yl, tetrazol-5-yl, 2-quinolyl, 3-quinolyl, 4-quinolyl, 2-quinazolyl, 4-quinazolyl, 2-quinoxalyl, 2-benzoxazolyl, 2-benzothiazolyl, benzimidazol-1-yl, benzimidazol-2-yl, indol-1-yl, indol-3-yl, 1H-indazol-3-yl, 1H-pyrrolo[2,3-b]pyrazin-2-yl, 1H-pyrrolo[2,3-b]pyridin-6-yl, 1H-imidazo[4,5-b]pyridin-2-yl, 1H-imidazo[4,5-c]pyridin-2-yl, 1H-imidazo[4,5-b]pyrazin-2-yl and the like; non-aromatic heterocyclic groups such as 1-pyrrolidinyl, piperidino, morpholino, thiomorpholino, 1-piperazinyl, hexamethylenimin-1-yl, oxazolidin-3-yl, thiazolidin-3-yl, imidazolidin-3-yl, 2-oxoimidazolidin-1-yl, 2,4-dioxoimidazolidin-3-yl, 2,4-dioxooxazolidin-3-yl, 2,4-dioxothiazolidin-3-yl and the like; and the like.

The heterocyclic group is preferably pyridyl, oxazolyl, thiazolyl, benzoxazolyl or benzothiazolyl.

### (1-3) Definition of substituent of hydrocarbon group and/or heterocyclic group of R¹

In the formula (I), the hydrocarbon group and heterocyclic group represented by R¹ each optionally have 1 to 5, preferably 1 to 3, substituents at substitutable positions. Examples of the substituent include optionally substituted aliphatic hydrocarbon group, optionally substituted alicyclic hydrocarbon group, optionally substituted aromatic hydrocarbon group, optionally substituted aromatic heterocyclic group, optionally substituted non-aromatic heterocyclic group, halogen atom, nitro group, optionally substituted amino group, optionally substituted acyl group, optionally substituted hydroxy group, optionally substituted thiol group, and optionally esterified or amidated carboxyl group.

Examples of the substituent of the "optionally substituted aliphatic hydrocarbon group, optionally substituted alicyclic hydrocarbon group, optionally substituted aromatic hydrocarbon group, optionally substituted aromatic heterocyclic group, optionally substituted non-aromatic heterocyclic group" include C₁₋₆ alkyl group, C₁₋₆ alkoxy group, halogen atom (e.g., fluorine, chlorine, bromine, iodine etc.), nitro group, C₁₋₆ haloalkyl group and C₁₋₆ haloalkoxy group. The number of the substituent is, for example, 1 to 3.

Examples of the aliphatic hydrocarbon group include linear or branched aliphatic hydrocarbon group having 1 to 15 carbon atoms, such as alkyl group, alkenyl group, alkynyl group and the like.

Preferable examples of the alkyl group include alkyl group having 1 to 10 carbon atoms, such as methyl, ethyl, propyl, isopropyl, butyl, isobutyl, sec.-butyl, t.-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

Preferable examples of the alkenyl group include alkenyl group having 2 to 10 carbon atoms, such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

Preferable examples of the alkynyl group include alkynyl group having 2 to 10 carbon atoms, such as ethynyl, 1-propinyl, 2-propinyl, 1-butynyl, 2-butynyl, 3-butynyl, 1-pentinyl, 2-pentinyl, 3-pentinyl, 4-pentinyl, 1-hexynyl, 2-hexynyl, 3-hexynyl, 4-hexynyl, 5-hexynyl, 1-heptinyl, 1-octynyl and the like.

Examples of the alicyclic hydrocarbon group include saturated or unsaturated alicyclic hydrocarbon group having 3 to 12 carbon atoms, such as cycloalkyl group, cycloalkenyl group, cycloalkadienyl group and the like.

Preferable examples of the cycloalkyl group include cycloalkyl group having 3 to 10 carbon atoms, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Preferable examples of the cycloalkenyl group include cycloalkenyl group having 3 to 10 carbon atoms, such as 2-cyclopenten-1-yl, 3-cyclopenten-1-yl, 2-cyclohexen-1-yl, 3-cyclohexen-1-yl and the like.

Preferable examples of the cycloalkadienyl group include cycloalkadienyl group having 4 to 10 carbon atoms, such as 2,4-cyclopentadien-1-yl, 2,4-cyclohexadien-1-yl, 2,5-cyclohexadien-1-yl and the like.

Preferable examples of the aromatic hydrocarbon group include aromatic hydrocarbon group having 6 to 14 carbon atoms (e.g., aryl group etc.), such as phenyl, naphthyl, anthryl, phenanthryl, acenaphtylenyl, biphenylyl and the like. Of these, phenyl, 1-naphthyl, 2-naphthyl and the like are preferable.

Preferable examples of the aromatic heterocyclic group include 5 to 7-membered aromatic monocyclic heterocyclic group containing, besides carbon atom, 1 to 4 heteroatoms selected from oxygen atom, sulfur atom and nitrogen atom, as a ring-constituting atom, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl and the like; bicyclic or tricyclic aromatic fused heterocycle having 3 to 13 carbon atoms, which contains, besides carbon atom, 1 to 5 heteroatoms selected from oxygen atom, sulfur atom and nitrogen atom, as a ring-constituting atom, such as benzofuranyl, isobenzofuranyl, benzo[b]thienyl, indolyl, isoindolyl, 1H-indazolyl, benzimidazolyl, benzoxazolyl, benzothiazolyl, 1H-benzotriazolyl, quinolyl, isoquinolyl, cinnolyl, quinazolyl, quinoxalinyl, phthalazinyl, naphthylidinyl, purinyl, pteridinyl, carbazolyl, α-carbonylyl, β-carbonylyl, γ-carbonylyl, acridinyl, phenoxazinyl, phenothiazinyl, phenazinyl, phenoxathiinyl, thianthrenyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like, and the like.

Preferable examples of the non-aromatic heterocyclic group include those having 2 to 10 carbon atoms, which contains, besides carbon atom, 1 to 3 heteroatoms selected from oxygen atom, sulfur atom and nitrogen atom, as a ring-constituting atom, such as oxiranyl, azetidinyl, oxetanyl, thietanyl, pyrrolidinyl, tetrahydrofuryl, tetrahydropyranyl, morpholinyl, thiomorpholinyl, piperazinyl, pyrrolidinyl, piperidino, morpholino, thiomorpholino and the like.

Examples of the halogen atom include fluorine, chlorine, bromine and iodine, of which fluorine and chlorine are preferable.

Examples of the optionally substituted amino group include amino group optionally mono- or di-substituted by alkyl group having 1 to 10 carbon atoms, cycloalkyl group having 3 to 10 carbon atoms, alkenyl group having 2 to 10 carbon atoms, cycloalkenyl group having 3 to 10 carbon atoms, acyl group having 1 to 13 carbon atoms (e.g., alkanoyl group having 2 to 10 carbon atoms, arylcarbonyl group having 7 to 13 carbon atoms etc.) or aryl group having 6 to 12 carbon atoms and the like. As used herein, the acyl group is defined as the optionally substituted acyl group to be mentioned below.

Examples of the substituted amino group include methylamino, dimethylamino, ethylamino, diethylamino, propylamino, dibutylamino, diallylamino, cyclohexylamino, acetylamino, propionylamino, benzoylamino, phenylamino, N-methyl-N-phenylamino and the like.

Examples of the acyl group of the optionally substituted acyl group include an acyl group having 1 to 13 carbon atoms, which is specifically formyl, a group wherein carbonyl group is bonded with alkyl group having 1 to 10 carbon atoms, cycloalkyl group having 3 to 10 carbon atoms, alkenyl group having 2 to 10 carbon atoms, cycloalkenyl group having 3 to 10 carbon atoms, aryl group having 6 to 12 carbon atoms or aromatic heterocyclic group (e.g., thienyl, furyl, pyridyl etc.), and the like.

Preferable examples of the acyl group include acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, isonicotinoyl and the like.

The acyl group may have 1 to 3 substituents at substitutable positions. Examples of such substituent include alkyl group having 1 to 3 carbon atoms, alkoxy group having 1 to 3 carbon atoms, halogen (e.g., fluorine, chlorine, iodine etc.), nitro, hydroxy, amino and the like.

The acyl group in a different form is represented by the following formula:

-COR¹¹, -SO₂R¹⁴, -SOR¹⁵ or -PO₃R¹⁶R¹⁷

wherein R¹¹, R¹⁴, R¹⁵, R¹⁶ and R¹⁷ are the same or different and each is optionally substituted hydrocarbon group.

Examples of the hydrocarbon group of the "optionally substituted hydrocarbon group" represented by R¹¹, R¹⁴, R¹⁵, R¹⁶ and R¹⁷ include alkyl group having 1 to 10 carbon atoms, cycloalkyl group having 3 to 10 carbon atoms, alkenyl group having 2 to 10 carbon atoms, cycloalkenyl group having 3 to 10 carbon atoms, and aryl group having 6 to 12 carbon atoms. Examples of the substituent of the aforementioned "optionally substituted hydrocarbon group" include C₁₋₆ alkyl group (except when hydrocarbon group is alkyl group), C₁₋₆ alkoxy group, halogen atom (e.g., fluorine, chlorine, bromine, iodine etc.), nitro group, C₁₋₆ haloalkyl group, C₁₋₆ haloalkoxy group. The number of substituents is, for example, 1 to 3.

In the optionally substituted hydroxy group, examples of the substituted hydroxy group include each optionally substituted alkoxy group, alkenyloxy group, aralkyloxy group, acyloxy group and aryloxy group and the like.

Preferable examples of the alkoxy group include alkoxy group having 1 to 10 carbon atoms, such as methoxy, ethoxy, propoxy, isopropoxy, butoxy, isobutoxy, sec.-butoxy, t.-butoxy, pentyloxy, isopentyloxy, neopentyloxy, hexyloxy, heptyloxy, nonyloxy, cyclobutoxy, cyclopentyloxy, cyclohexyloxy and the like.

Preferable examples of the alkenyloxy group include alkenyloxy group having 2 to 10 carbon atoms, such as allyloxy, crotyloxy, 2-pentenyloxy, 3-hexenyloxy, 2-cyclopentenylmethoxy, 2-cyclohexenylmethoxy and the like.

Preferable examples of the aralkyloxy group include aralkyloxy group having 7 to 10 carbon atoms, such as phenyl-C₁₋₄ alkyloxy (e.g., benzyloxy, phenethyloxy etc.) and the like.

Preferable examples of the acyloxy group include.acyloxy group having 2 to 13 carbon atoms, more preferably alkanoyloxy having 2 to 4 carbon atoms (e.g., acetyloxy, propionyloxy, butyryloxy, isobutyryloxy etc.) and the like.

Preferable examples of the aryloxy group include aryloxy group having 6 to 14 carbon atoms, such as phenoxy, naphthyloxy and the like.

The above-mentioned alkoxy group, alkenyloxy group, aralkyloxy group, acyloxy group and aryloxy group may have 1 or 2 substituents at substitutable positions. Examples of the substituent include halogen (e.g., fluorine, chlorine, bromine etc.), alkoxy group having 1 to 3 carbon atoms and the like. Examples of the substituted aryloxy group include 4-chlorophenoxy, 2-methoxyphenoxy and the like.

In the optionally substituted thiol group, examples of the substituted thiol group include alkylthio, cycloalkylthio, aralkylthio, acylthio, arylthio, heteroarylthio and the like.

Preferable examples of the alkylthio group include alkylthio group having 1 to 10 carbon atoms, such as methylthio, ethylthio, propylthio, isopropylthio, butylthio, isobutylthio, sec.-butylthio, t.-butylthio, pentylthio, isopentylthio, neopentylthio, hexylthio, heptylthio, nonylthio and the like.

Preferable examples of the cycloalkylthio group include cycloalkylthio group having 3 to 10 carbon atoms, such as cyclobutylthio, cyclopentylthio, cyclohexylthio and the like.

Preferable examples of the aralkylthio group include aralkylthio group having 7 to 10 carbon atoms, such as phenyl-C₁₋₄ alkylthio (e.g., benzylthio, phenethylthio etc.) and the like.

Preferable examples of acylthio group include acylthio group having 2 to 13 carbon atoms, more preferably alkanoylthio group having 2 to 4 carbon atoms (e.g., acetylthio, propionylthio, butyrylthio, isobutyrylthio etc.) and the like.

Preferable examples of arylthio group include arylthio group having 6 to 14 carbon atoms, such as phenylthio, naphthylthio and the like.

Preferable examples of heteroarylthio group include 2-pyridylthio, 3-pyridylthio and the like, as well as 2-imidazolylthio, 1,2,4-triazol-5-ylthio and the like.

The above-mentioned alkylthio group, cycloalkylthio group, aralkylthio group, acylthio group, arylthio group and heteroarylthio group may have 1 or 2 substituents at substitutable positions. Examples of such substituent include halogen (e.g., fluorine, chlorine, bromine etc.), alkoxy group having 1 to 3 carbon atoms and the like.

In the optionally esterified carboxyl group, examples of the esterified carboxyl group include alkoxycarbonyl group having 2 to 5 carbon atoms (e.g., methoxycarbonyl, ethoxycarbonyl, propoxycarbonyl, butoxycarbonyl etc.), aralkyloxycarbonyl group having 8 to 10 carbon atoms (e.g., benzyloxycarbonyl etc.), aryloxycarbonyl group having 7 to 15 carbon atoms optionally substituted by 1 or 2 alkyl groups having 1 to 3 carbon atoms (e.g., phenoxycarbohyl, p-tolyloxycarbonyl etc.) and the like.

In the optionally amidated carboxyl group, examples of the amidated carboxyl group include a group represented by the formula: -CON(R¹²) (R¹³)
wherein R¹² and R¹³ are the same or different and each is hydrogen atom, optionally substituted hydrocarbon group or optionally substituted heterocyclic group.

Here, examples of the hydrocarbon group of the "optionally substituted hydrocarbon group" and the heterocyclic group of the "optionally substituted heterocyclic group" represented by R¹² and R¹³ include aliphatic hydrocarbon group, alicyclic hydrocarbon group, aromatic hydrocarbon group and heterocyclic group exemplified for the above-mentioned the "hydrocarbon group of the "optionally substituted hydrocarbon group represented by R¹"" and "the heterocyclic group of the "optionally substituted heterocyclic group represented by R¹"". The hydrocarbon group and heterocyclic group may have 1 to 3 substituents at substitutable positions, and examples of such substituent include halogen (e.g., fluorine, chlorine, bromine, iodine etc.), alkyl having 1 to 4 carbon atoms, alkoxy group having 1 to 4 carbon atoms and the like.

In the formula (I), the substituent of the hydrocarbon group and heterocyclic group represented by R¹ is preferably alkyl group having 1 to 10 carbon atoms, aromatic heterocyclic group and aryl group having 6 to 14 carbon atoms, more preferably alkyl having 1 to 3 carbon atoms, furyl, thienyl, phenyl and naphthyl.

The substituent of the hydrocarbon group and heterocyclic group represented by R¹, when it is alicyclic hydrocarbon group, aromatic hydrocarbon group, aromatic heterocyclic group or non-aromatic heterocyclic group, may each have one or more, preferably 1 to 3, suitable substituents. Examples of such substituent include alkyl group having 1 to 6 carbon atoms, alkenyl group having 2 to 6 carbon atoms, cycloalkyl group having 3 to 10 carbon atoms, aryl group having 6 to 14 carbon atoms (e.g., phenyl, naphthyl etc.), aromatic heterocyclic group (e.g., thienyl, furyl, pyridyl, oxazolyl, thiazolyl etc.), non-aromatic heterocyclic group (e.g., tetrahydrofuryl, morpholino, thiomorpholino, piperidino, pyrrolidinyl, piperazinyl etc.), aralkyl group having 7 to 9 carbon atoms, amino group, amino group mono- or di-substituted by alkyl group having 1 to 4 carbon atoms or acyl group having 2 to 8 carbon atoms (e.g., alkanoyl group etc.), amidino group, acyl group having 2 to 8 carbon atoms (e.g., alkanoyl group etc.), carbamoyl group, carbamoyl group mono- or di-substituted by alkyl group having 1 to 4 carbon atoms, sulfamoyl group, sulfamoyl group mono- or di-substituted by alkyl group having 1 to 4 carbon atoms, carboxyl group, alkoxycarbonyl group having 2 to 8 carbon atoms, hydroxy group, alkoxy group having 1 to 6 carbon atoms, alkenyloxy group having 2 to 5 carbon atoms, cycloalkyloxy group having 3 to 7 carbon atoms, aralkyloxy group having 7 to 9 carbon atoms, aryloxy group having 6 to 14 carbon atoms (e.g., phenyloxy, naphthyloxy etc.), thiol group, alkylthio group having 1 to 6 carbon atoms, aralkylthio group having 7 to 9 carbon atoms, arylthio group having 6 to 14 carbon atoms (e.g., phenylthio, naphthylthio etc.), sulfo group, cyano group, azide group, nitro group, nitroso group, halogen atom (e.g., fluorine, chlorine, bromine, iodine) and the like.

### (1-4) Preferable example of R¹

In the formula (I), R¹ is preferably optionally substituted heterocyclic group, more preferably optionally substituted pyridyl, optionally substituted oxazolyl, optionally substituted thiazolyl or optionally substituted triazolyl. R¹ is particularly preferably pyridyl, oxazolyl, thiazolyl or triazolyl each optionally having 1 or 2 substituents selected from alkyl having 1 to 3 carbon atoms, cycloalkyl having 3 to 7 carbon atoms, furyl, thienyl, phenyl and naphthyl. Here, furyl, thienyl, phenyl and naphthyl may have 1 or 2 substituents selected from alkyl having 1 to 3 carbon atoms, alkoxy having 1 to 3 carbon atoms, halogen (e.g., fluorine, chlorine, bromine, iodine etc.) and haloalkyl having 1 to 3 carbon atoms.

Preferable examples of R¹ include optionally substituted heterocyclic group and optionally substituted cyclic hydrocarbon group represented by the following formulas:

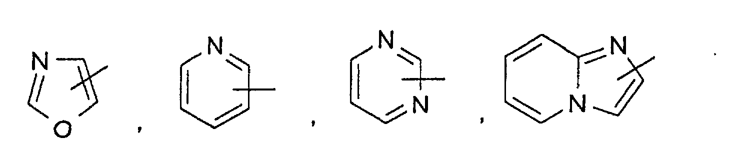

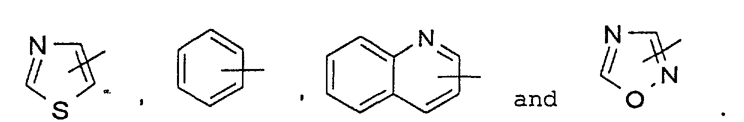

These groups optionally have 1 or 2 substituents selected from phenyl, furyl, thienyl and alkyl having 1 to 4 carbon atoms. The phenyl, furyl and thienyl may have 1 or 2 substituents selected from alkyl having 1 to 6 carbon atoms, alkoxy having 1 to 6 carbon atoms, halogen (e.g., fluorine, chlorine, bromine, iodine etc.), nitro, haloalkyl having 1 to 6 carbon atoms and haloalkoxy having 1 to 6 carbon atoms. The alkyl having 1 to 4 carbon atoms may have 1 or 2 substituents selected from alkoxy having 1 to 6 carbon atoms, halogen (e.g., fluorine, chlorine, bromine, iodine etc.), nitro, haloalkyl having 1 to 6 carbon atoms and haloalkoxy having 1 to 6 carbon atoms.

R¹ is more preferably a group represented by the following formula 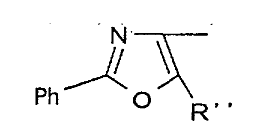 wherein Ph is an optionally substituted phenyl group, R" is a hydrogen atom or an optionally substituted alkyl group having 1 to 6 carbon atoms.

Examples of the substituent of phenyl group represented by Ph and alkyl group having 1 to 6 carbon atoms, which is represented by R", include alkoxy having 1 to 6 carbon atoms, halogen (e.g., fluorine, chlorine, bromine, iodine etc.), nitro, haloalkyl having 1 to 6 carbon atoms and haloalkoxy having 1 to 6 carbon atoms. The number of substituents is, for example, 1 to 3.

### (2) Definition of X

In the formula (I), X is a bond or a group represented by -CO-, -CH(OH)- or -NR⁶- (R⁶ is hydrogen atom or optionally substituted alkyl group), preferably a bond, -CH(OH)- or -NR⁶-, more preferably a bond or -NR⁶-.

As used herein, examples of the alkyl group of the "optionally substituted alkyl group", which is represented by R⁶, include, alkyl having 1 to 4 carbon atoms, such as methyl, ethyl, propyl, isopropyl, butyl, isobutyl, sec.-butyl, t.-butyl and the like. The alkyl group may have 1 to 3 substituents at substitutable positions. Examples of the substituent include halogen (e.g., fluorine, chlorine, bromine, iodine), alkoxy group having 1 to 4 carbon atoms (e.g., methoxy, ethoxy, propoxy, isopropoxy, butoxy, isobutoxy, sec.-butoxy, t.-butoxy etc.), hydroxy group, nitro group, acyl group having 1 to 4 carbon atoms (e.g., alkanoyl group having 1 to 4 carbon atoms such as formyl, acetyl, propionyl etc.).

### (3) Definition of n and Y

In the formula (I), n is an integer of 1 to 3, preferably 1 or 2.

In the formula (I), Y is -O-, -S-, -SO-, -SO₂- or -NR⁷- (R⁷ is hydrogen atom or optionally substituted alkyl group), which is preferably -O-, -S- or -NR⁷-. Here, examples of "optionally substituted alkyl group" represented by R⁷ include those similar to the above-mentioned "optionally substituted alkyl group" represented by R⁶.

### (4) Definition of ring A

In the formula (I), ring A is benzene ring, wherein the benzene ring optionally having 1 to 3 additional substituent(s) at substitutable positions. Examples of the substituent include alkyl group, optionally substituted hydroxy group, halogen atom, optionally substituted acyl group, nitro group and optionally substituted amino group. As these, those exemplified as the substituent of the hydrocarbon group and heterocyclic group represented by R¹ can be used.

The substituent is preferably alkyl having 1 to 4 carbon atoms, alkoxy group having 1 to 4 carbon atoms or halogen atom.

In the formula (I), a partial structural formula 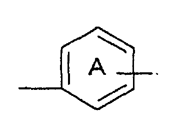 is preferably

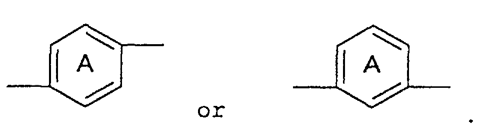

### (5) Definition of p

In the formula (I), p is an integer of 1 to 8, preferably an integer of 1 to 3.

### (6) Definition of R²

In the formula (I), examples of the "optionally substituted hydrocarbon group" represented by R² include those exemplified as the "optionally substituted hydrocarbon group" represented by R¹.

Examples of the "optionally substituted heterocyclic group" represented by R² include those exemplified as the "optionally substituted heterocyclic group" represented by R¹.

In the formula (I), R² is preferably optionally substituted hydrocarbon group. R² is more preferably aliphatic hydrocarbon group, alicyclic hydrocarbon group, aromatic-aliphatic hydrocarbon group or aromatic hydrocarbon group, all of which may be substituted, particularly preferably alkyl group having 1 to 4 carbon atoms, phenylalkenyl group having 8 to 10 carbon atoms or aryl group having 6 to 14 carbon atoms, all of which may be substituted.

The substituent these hydrocarbon groups may have is preferably halogen atom, alkoxy group having 1 to 4 carbon atoms, aryloxy group having 6 to 14 carbon atoms or aromatic heterocyclic group (e.g., furyl, thienyl). The number of substituents is, for example, 1 to 3.

### (7) Definition of q and m

In the formula (I), q is an integer of 0 to 6, preferably 0 to 4. m is 0 or 1.

### (8) Definition of R³

R³ is hydroxy group, -OR⁸ (R⁸ is optionally substituted hydrocarbon group) or -NR⁹R¹⁰ (R⁹ and R¹⁰ are the same or different and each is hydrogen atom, optionally substituted hydrocarbon group, optionally substituted heterocyclic group or optionally substituted acyl group, and R⁹ and R¹⁰ may be bonded to form a ring).

In the formula (I), examples of the "optionally substituted hydrocarbon group" represented by R⁸ include those exemplified as the "optionally substituted hydrocarbon group" represented by R¹. Preferably, R⁸ is "alkyl group having 1 to 4 carbon atoms" or "aryl group having 6 to 10 carbon atoms optionally substituted by alkyl group having 1 to 4 carbon atoms or halogen atom". As used herein, examples of the aforementioned alkyl group having 1 to 4 carbon atoms include methyl, ethyl, propyl, butyl, isobutyl, sec-butyl and t-butyl, particularly preferably methyl and ethyl. Examples of the halogen of the "aryl group having 6 to 10 carbon atoms optionally substituted by alkyl group having 1 to 4 carbon atoms or halogen atom" include fluorine, chlorine, bromine, iodine, particularly preferably chlorine. Examples of the aryl group having 6 to 10 carbon atoms include phenyl and naphthyl, particularly preferably phenyl.

Examples of the "optionally substituted hydrocarbon group" and "optionally substituted heterocyclic group" represented by R⁹ and R¹⁰ include those similar to the "optionally substituted hydrocarbon group" and "optionally substituted heterocyclic group" represented by R¹.

Examples of the "optionally substituted acyl group" represented by R⁹ and R¹⁰ include those similar to the "optionally substituted acyl group" exemplified as the substituent that the "optionally substituted hydrocarbon group" represented by R¹ may possess.

R⁹ and R¹⁰ may be bonded to form a 5 to 7-membered cyclic amino group. Examples of specific cyclic amino group include 1-pyrrolidinyl, 1-piperidinyl, 1-hexamethyleniminyl, 4-morpholino, 4-thiomorpholino and the like.

### (9) Definition of R⁴ and R⁵

In the formula (I), R⁴ and R⁵ are the same or different and each is hydrogen atom or optionally substituted hydrocarbon group, and R⁴ and R² may be bonded to form a ring.

In the formula (I), examples of the "optionally substituted hydrocarbon group" represented by R⁴ and R⁵ include those similar to the aforementioned "optionally substituted hydrocarbon group" represented by R¹, preferably those similar to the aforementioned "optionally substituted alkyl group" represented by R⁶ and the like.

In the formula (I), R⁴ may be bonded to R² to form a ring. Examples of the ring formed by bonding of R⁴ and R² include cycloalkane having 5 to 11 carbon atoms and cycloalkene having 5 to 11 carbon atoms and the like, which is specifically cyclopentane, cyclopentene, cyclohexane, cyclohexene, cycloheptane, cycloheptene, cyclooctane, cyclooctene, cyclononane, cyclononene, cyclodecane, cyclodecene, cycloundecane and cycloundecene and the like.

### (10) (E) form and/or (Z) form compound(s)

The compound represented by the formula (I) includes (E) form and (Z) form around an imino bond. The compound includes these (E) form and (Z) form as single compounds, and a mixture thereof.

### (11) Preferable examples

Preferable examples of the compounds represented by the formula (I) include the compounds represented by the following formula: 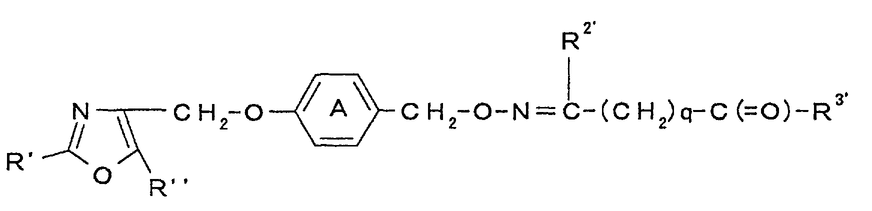 wherein
R' is a phenyl, a furyl or a thienyl group each optionally substituted by 1 or 2 substituents selected from alkyl having 1 to 6 carbon atoms, alkoxy having 1 to 6 carbon atoms, halogen (e.g., fluorine, chlorine, bromine, iodine etc.), nitro, haloalkyl having 1 to 6 carbon atoms and haloalkoxy having 1 to 6 carbon atoms;
R" is hydrogen atom or optionally substituted alkyl having 1 to 6 carbon atoms (more preferably hydrogen atom, methyl group or ethyl group);
R^{2'} is a hydrogen atom, a phenyl group optionally substituted by at least one substituent selected from alkyl having 1 to 6 carbon atoms, alkoxy having 1 to 6 carbon atoms and halogen;
q is an integer of 1 to 6;
R^{3'} is a hydroxy, an alkoxy having 1 to 6 carbon atoms, or a group represented by -NR⁹R¹⁰ (R⁹ and R¹⁰ are the same or different and each is hydrogen atom, optionally substituted hydrocarbon group, optionally substituted heterocyclic group, optionally substituted acyl group, or R⁹ and R¹⁰ may be bonded to form a ring); and
ring A is an optionally substituted benzene ring, or a salt thereof.

Preferable examples of the compounds represented by the formula (I) also include the compounds represented by the following formula: 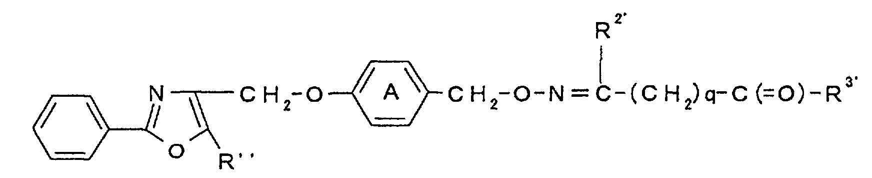 wherein each symbol is as defined above, or a salt thereof.

Preferable examples of the compounds represented by the formula (I) are the following compounds (1)-(10) and the like.
(1) Z-2-[4-(5-methyl-2-phenyl-4-oxazolylmethoxy)benzyloxyimino]-2-phenylacetic acid
(2) Z-4-[4-(5-methyl-2-phenyl-4-oxazolylmethoxy)benzyloxyimino]-4-phenylbutyric acid
(3) Z-2-(4-bromophenyl)-2-[4-(5-methyl-2-phenyl-4-oxazolylmethoxy)benzyloxyimino]acetic acid
(4) Z-2-[4-(5-methyl-2-phenyl-4-oxazolylmethoxy)benzyloxyimino]-2-(4-phenoxyphenyl)acetic acid
(5) Z-4-(4-fluorophenyl)-4-[4-(5-methyl-2-phenyl-4-oxazolylmethoxy)benzyloxyimino]butyric acid
(6) Z-3-methyl-2-[4-(5-methyl-2-phenyl-4-oxazolylmethoxy)-benzyloxyimino]butyric acid
(7) E-4-[4-(5-methyl-2-phenyl-4-oxazolylmethoxy)benzyloxyimino]-4-phenylbutyric acid
(8) E-4-(4-fluorophenyl)-4-[4-(5-methyl-2-phenyl-4-oxazolylmethoxy)benzyloxyimino]butyric acid
(9) E-4-[4-(5-methyl-2-phenyl-4-oxazolylmethoxy)-benzyloxyimino]-4-phenylbutylamide
(10) E-8-[4-(5-methyl-2-phenyl-4-oxazolylmethoxy)benzyloxyimino]-8-phenyloctanoic acid

These compounds are sometimes to be abbreviated in the following as compound (1), compound (2) and the like.

The salt of the compound represented by the formula (I) (hereinafter sometimes to be simply abbreviated as compound (I)) is preferably a pharmacologically acceptable salt, for example, salt with inorganic base, salt with organic base, salt with inorganic acid, salt with organic acid, salt with basic or acidic amino acid and the like.

Preferable examples of the salt with inorganic base include alkali metal salts, such as sodium salt, potassium salt and the like; alkaline earth metal salts, such as calcium salt, magnesium salt and the like; aluminum salt, ammonium salt and the like.

Preferable examples of the salts with organic base include salts with trimethylamine, triethylamine, pyridine, picoline, ethanolamine, diethanolamine, triethanolamine, dicyclohexylamine, N,N-dibenzylethylenediamine and the like.

Preferable examples of the salt with inorganic acid include salts with hydrochloric acid, hydrobromic acid, nitric acid, sulfuric acid, phosphoric acid and the like.

Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with basic amino acid include salts with arginine, lysin, ornithine and the like and preferable examples of the salt with acidic amino acid include salts with aspartic acid, glutamic acid and the like.

Of the above-mentioned salts, sodium salt, potassium salt, hydrochloride and the like are preferable.

### (12) Production method

Compound (I) can be produced by, for example, the method described in JP-A-2000-34266 (JP application No. 11-130543, WO99/58510). As such method, for example, the following production method is mentioned. 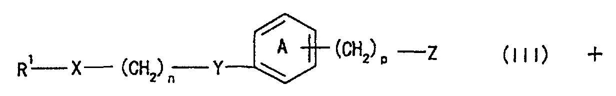 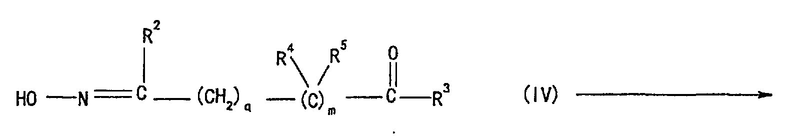 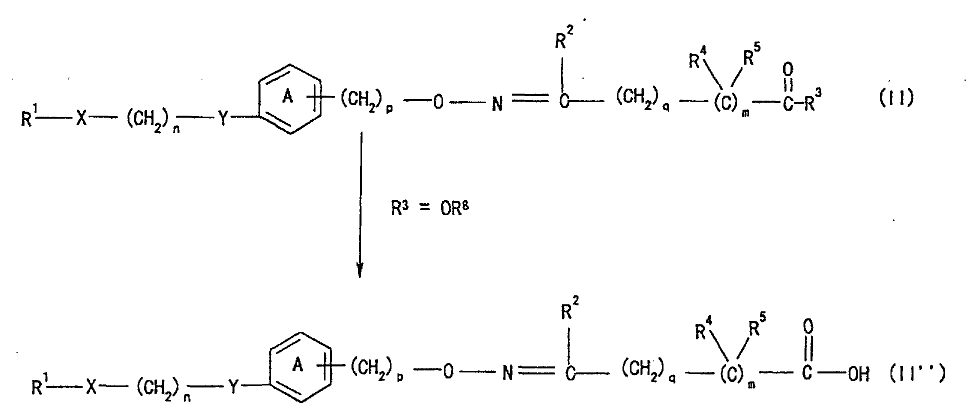 wherein Z is a hydroxy group, a halogen atom or a group represented by OSO₂R¹⁸ (R¹⁸ is alkyl group having 1 to 4 carbon atoms, aryl group having 6 to 10 carbon atoms optionally substituted by alkyl group having 1 to 4 carbon atoms), and other symbols are as defined above.

Here, examples of the alkyl group having 1 to 4 carbon atoms of the "alkyl group having 1 to 4 carbon atoms" and "aryl group having 6 to 10 carbon atoms optionally substituted by alkyl group having 1 to 4 carbon atoms" represented by R¹⁸ include methyl, ethyl, propyl, isopropyl, butyl, isobutyl, sec.-butyl and t.-butyl, particularly preferably methyl.

Examples of the aryl group having 6 to 10 carbon atoms of the "aryl group having 6 to 10 carbon atoms optionally substituted by alkyl group having 1 to 4 carbon atoms" represented by R¹⁸ include phenyl and naphthyl, particularly preferably phenyl.

According to this method, compound (III) and compound (IV) are reacted to produce compound (II).

When Z is a hydroxy group, this reaction is carried out according to the method known per se, such as the method described in Synthesis, page 1 (1981), or a method analogous thereto. That is, this reaction is generally carried out in the presence of an organic phosphorus compound and electrophile in a solvent that does not adversely affect the reaction.

Examples of the organic phosphorus compound include triphenylphosphine, tributylphosphine and the like.

Examples of the electrophile include diethyl azodicarboxylate, diisopropyl azodicarboxylate, azodicarbonyldipiperazine and the like.

The amount of the organic phosphorus compound and the electrophile to be used is preferably 1-5 molar equivalent amount relative to compound (IV).

Examples of the solvent that does not adversely affect the reaction include ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethyl sulfoxide and the like; and the like. These solvents may be admixed at a suitable ratio for use.

The reaction temperature is generally -50°C to 150°C, preferably -10°C to 100°C.

The reaction time is 0.5 to 20 hours.

When Z is halogen atom or a group represented by OSO₂R¹⁸, this reaction is carried out in the presence of a base in a solvent that does not adversely affect the reaction according to a conventional method.

Examples of the base include alkali metal salts such as potassium hydroxide, sodium hydroxide, sodium hydrogencarbonate, potassium carbonate and the like; amines such as pyridine, triethylamine, N,N-dimethylaniline, 1,8-diazabicyclo[5.4.0]undeca-7-ene and the like; metal hydrides such as potassium hydride, sodium hydride and the like; and alkali metal alkoxides such as sodium methoxide, sodium ethoxide, potassium t.-butoxide and the like.

The amount of these bases to be used is preferably 1-5 molar equivalents relative to compound (IV).

Examples of the solvent that does not adversely affect the reaction include aromatic hydrocarbons such as benzene, toluene, xylene and the like; ethers such as tetrahydrofuran, dioxane and the like; ketones such as acetone, 2-butanone and the like; halogenated hydrocarbons such as chloroform, dichloromethane and the like; amides such as N,N-dimethylformamide and the like; sulfoxides such as dimethyl sulfoxide and the like; and the like. These solvents may be admixed at a suitable ratio for use.

The reaction temperature is generally -50 to 150°C, preferably -10°C to 100°C.

The reaction time is generally 0.5 to 20 hours.

Where desired, compound (II, R³ = OR⁸) is subjected to hydrolysis reaction to produce compound (II").

This hydrolysis reaction is carried out in the presence of acid or base in an aqueous solvent according to a conventional method.

Examples of the acid include hydrochloric acid, sulfuric acid, acetic acid, hydrobromic acid and the like.

Examples of the base include alkali metal carbonates such as potassium carbonate, sodium carbonate and the like; alkali metal alkoxides such as sodium methoxide and the like; alkali metal hydroxides such as potassium hydroxide, sodium hydroxide, lithium hydroxide and the like; and the like.

The amount of the acid or the base to be used is generally an excess amount over compound (II). Preferably, the amount of the acid to be used is 2-50 equivalents relative to compound (II), and the amount of the base to be used is 1.2-5 equivalents relative to compound (II).

Examples of the aqueous solvent include a mixed solvent of water with one or more solvents selected from alcohols such as methanol, ethanol and the like; ethers such as tetrahydrofuran, dioxane and the like; dimethyl sulfoxide, acetone and the like, and the like.

The reaction temperature is generally -20°C to 150°C, preferably -10°C to 100°C.

The reaction time is generally 0.1 to 20 hours.

The compounds (II) and (II") thus obtained can be isolated and purified according to known separation and purification means, such as concentration, concentration under reduced pressure, solvent extraction, crystallization, recrystallization, phase transfer, chromatography and the like.

The compound (III) and compound (IV) to be used as a starting material compound in the above-mentioned production method are known compounds and compound (III) wherein Z is hydroxy group is described in, for example, EP-A-710659. The compound (III) is described in EP-A-629624 (JP-A-7-53555), WO 98/03505 and the like. The compound (III) can be also produced by a method analogous to methods described in these publications.

The compound (IV) is described in, for example, Journal fur Praktische Chemie, vol. 311, 370 (1969); Canadian Journal of Chemistry, vol. 48, 1948 (1970); Journal of Heterocyclic Chemistry, vol. 25, 1283 (1988); and the like. The compound (IV) can be also produced by a method analogous to methods described in these publications.

In the present invention, the PPARδ agonist-like substance and the PPARγ agonist-like substance are not limited by the above-mentioned exemplification. As long as a similar effect is achieved, any substance can be used. In addition, these may further have a PPARα function modulating action (agonist or antagonist activity).

In consideration of the burden on the patients to be administration targets, troubles of preparing medicine and the like in the present invention, a single substance desirably simultaneously has both a PPARδ agonist-like action and a PPARγ agonist-like action. Such substance is exemplified by the above-mentioned compound (I), YM-16638 (mentioned above), p-[3-(4-acetyl-3-hydroxy-2-propylphenoxy)propoxy]phenylbutyric acid (mentioned above), 5-[3-[4-[(5-methyl-2-phenyl-1,3-thiazol-4-yl)methoxy]phenyl]propyl]-1,3-oxazolidine-2,4-dione (mentioned above) and the like.

A pharmaceutical agent containing both a PPARδ agonist-like substance and a PPARγ agonist-like substance is also one embodiment of the present invention. Examples of such preferable combination include a PPARγ agonist such as the aforementioned insulin sensitizers (e.g., troglitazone, rosiglitazone, englitazone, ciglitazone, pioglitazone etc.) and the aforementioned carbaprostacyclin (cPGI), L-165041 or compound (I) and the like.

Since compound (I) itself has both a PPARδ agonist-like action and a PPARγ agonist-like action, this can be also used in combination with an insulin sensitizer.

The dose of the agent of the present invention when the PPARγ agonist-like substance and the PPARδ agonist-like substance are different substances needs only to be within the effective amount of the respective substances, and when the PPARγ agonist-like substance and the PPARδ agonist-like substance are the same, it is within the effective amount of the substance.

The dose when the agent of the present invention is orally administered to, for example, adult diabetic patients (body weight 60 kg) is, for example, about 0.1 to about 600 mg/day, preferably about 12 to about 240 mg/day, in terms of the PPARγ agonist-like substance or PPARδ agonist-like substance. The dose may be administered once a day or divided and given in 2 or 3 portions.

The agent of the present invention has low toxicity and can be used as an agent for the prophylaxis or treatment of various diseases to be mentioned below in mammals (e.g., human, mouse, rat, rabbit, dog, cat, bovine, horse, pig, monkey etc.).

The agent of the present invention may contain a pharmacologically acceptable carrier. As such carrier, various organic or inorganic carrier substances conventionally used as starting materials for preparation of medicine are used in the form of excipient, lubricant, binder or disintegrant for solid preparations; solvent, dissolution aids, suspending agent, isotonicity agent, buffering agent or soothing agent for liquid preparations; and the like. Where necessary, additives for preparation, such as preservative, antioxidant, coloring agent, sweetening agent and the like can be also used.

Preferable examples of the excipient include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, dextrin, pullulan, light silicic anhydride, synthetic aluminum silicate, magnesium aluminometasilicate and the like.

Preferable examples of the lubricant include magnesium stearate, calcium stearate, talc, colloidal silica and the like.

Preferable examples of the binder include pregelatinized starch, saccharose, gelatin, gum arabic, methyl cellulose, carboxymethyl cellulose, carboxymethylcellulose sodium, crystalline cellulose, sucrose, D-mannitol, trehalose, dextrin, pullulan, hydroxypropyl cellulose, hydroxypropylmethyl cellulose, polyvinylpyrrolidone and the like.

Preferable examples of disintegrant include lactose, sucrose, starch, carboxymethyl cellulose, carboxymethylcellulose calcium, crosscarmellose sodium, carboxymethyl starch sodium, light silicic anhydride, low-substituted hydroxypropyl cellulose and the like.

Preferable examples of the solvent include water for injection, physiological brine, Ringer's solution, alcohol, propylene glycol, polyethylene glycol, sesame oil, corn oil, olive oil, cotton oil and the like.

Preferable examples of dissolution aids include polyethylene glycol, propylene glycol, D-mannitol, trehalose, benzyl benzoate, ethanol, tris aminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, laurylaminopropionic acid, lecithin, benzalkonium chloride, benzethonium chloride, glyceryl monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; polysorbates, polyoxyethylene hydrogenated castor oil and the like.

Preferable examples of the isotonicity agent include sodium chloride, glycerin, D-mannitol, D-sorbitol, glucose and the like.

Preferable examples of the buffering agent include buffers such as phosphate, acetate, carbonate, citrate and the like, and the like.

Preferable examples of the soothing agent include benzyl alcohol and the like.

Preferable examples of the preservative include p-oxybenzoic acid esters, chlorobutanol, benzyl alcohol, phenethyl alcohol, dehydroacetic acid, sorbic acid and the like.

Preferable examples of the antioxidant include sulfite, ascorbate and the like.

Preferable examples of the coloring agent include water-soluble food tar dye (e.g., foodcolors such as Food Color Red No. 2 and No. 3, Food Color yellow No. 4 and No, 5, Food Color Blue No. 1 and No. 2 and the like, water-insoluble rake dye (e.g., aluminum salt of the aforementioned water-soluble food tar dye etc.), natural color (e.g., β-carotene, chlorophyll, red ferric oxide etc.) and the like.

Preferable examples of the sweetening agent include saccharin sodium, dipotassium glycyrrhizinate, aspartame, stevia and the like.

When the PPARδ agonist-like substance and the PPARγ agonist-like substance are different substances, the agent of the present invention may be a single preparation containing both substances or two different kinds of preparations containing either of the substances. In this case, the dosage forms of the preparations do not need to be the same. The dosage form typically employed for medical treatment is appropriately selected according to the active ingredient of these preparations.

Examples of the dosage form of the agent of the present invention include oral agents such as tablet, capsule (including soft capsule and microcapsule), granule, powder, syrup, emulsion, suspension and the like; and parenteral agents such as injection (e.g., subcutaneous injection, intravenous injection, intramuscular injection, intraperitoneal injection etc.), external preparation (e.g., preparation for nasal administration, transdermal preparation, ointment etc.), suppository (e.g., rectal suppository, vaginal suppository etc.), pellet, drop and sustained release preparation, all of which can be orally or parenterally administered safely.

The agent of the present invention can be produced according to a method conventional in the technical field of preparing medicine, for example, the method described in Japan Pharmacopoeia and the like. A specific production method of a preparation is explained in detail in the following.

For example, oral agents can be produced by adding, for example, excipient (e.g., lactose, sucrose, starch, D-mannitol etc.), disintegrant (e.g., carboxymethylcellulose calcium etc.), binder (e.g., pregelatinized starch, gum arabic, carboxymethylcellulose, hydroxypropylcellulose, polyvinylpyrrolidone etc.) or lubricant (e.g., talc, magnesium stearate, polyethylene glycol 6000 etc.) and the like to the active ingredient, compression-molding the mixture, and where necessary, coating with a coating material for the purpose of masking of taste, enteric property or sustained release property by a method known *per se.*

Examples of the coating material include sugar coating material, water-soluble film coating material, enteric film coating material, sustained release film coating material and the like.

As the sugar coating material, sucrose is used, and one or two kinds selected from talc, precipitated calcium carbonate, gelatin, gum arabic, pullulan, carnauba wax and the like may be used in combination.

Examples of the water-soluble film coating material include cellulose polymers such as hydroxypropylcellulose, hydroxypropylmethylcellulose, hydroxyethylcellulose, methylhydroxyethylcellulose and the like; synthetic polymers such as polyvinylacetaldiethylaminoacetate, aminoalkylmethacrylate copolymer E [Eudragit E (trademark), Röhm Pharma], polyvinylpyrrolidone and the like; polysaccharides such as pullulan and the like; and the like.

Examples of the enteric film coating material include cellulose polymers such as hydroxypropylmethylcellulose phthalate, hydroxypropylmethylcellulose acetate succinate, carboxymethylethylcellulose, cellulose acetate phthalate and the like; acrylic acid polymers such as methacrylic acid copolymer L [Eudragit L (trademark), Röhm Pharma], methacrylic acid copolymer LD [Eudragit L-30D55 (trademark), Röhm Pharma], methacrylic acid copolymer S [Eudragit S (trademark), Röhm Pharma] and the like; naturally occurring substances such as shellac and the like; and the like.

Examples of the sustained release film coating material include cellulose polymers such as ethylcellulose and the like; acrylic acid polymers such as aminoalkylmethacrylate copolymer RS [Eudragit RS (trade name), Röhm Pharma], ethyl acrylate methyl methacrylate copolymer suspension [Eudragit NE (trade name), Röhm Pharma] and the like; and the like.

The above-mentioned coating materials may be used in combination of two or more kinds thereof admixed at a suitable proportions. For coating, for example, shading agents such as titanium oxide, iron sesquioxide and the like may be used.

Injections can be produced by dissolving, suspending or emulsifying the active ingredient along with a dispersant (e.g., polysorbate 80, polyoxyethylene hydrogenated castor oil 60 etc.), polyethylene glycol, carboxymethylcellulose, sodium alginate and etc.), a preservative (e.g., methylparaben, propylparaben, benzyl alcohol, chlorobutanol, phenol etc.), an isotonicity agent (e.g., sodium chloride, glycerin, D-mannitol, D-sorbitol, glucose etc.) and the like in an aqueous solvent (e.g., distilled water, physiological saline, Ringer's solution etc.) or an oily solvent (e.g., vegetable oils such as olive oil, sesame oil, cotton oil, corn oil etc., propylene glycol etc.) and the like. Where desired, additives such as dissolution aids (e.g., sodium salicylate, sodium acetate etc.), stabilizers (e.g., human serum albumin etc.), soothing agents (e.g., benzyl alcohol etc.) and the like may be used.

Of the aforementioned various dosage forms, particularly oral agents such as tablet, capsule and the like are preferable in view of convenience during administration.

The agent of the present invention is effective for the suppression of body weight gain observed in patients with various diseases (e.g., diabetes), who are under medication of PPARγ agonist-like substance (e.g., insulin sensitizer), and is useful for the treatment or prophylaxis of PPARδ-related diseases (e.g., hypercholesterolemia, hypo-high-density-lipoproteinemia) and the like.

The agent of the present invention is capable of suppressing body weight gain caused by PPARγ agonist-like substances, which is observed in, for example, patients (e.g., diabetic patients) under medication of a PPARγ agonist-like substance, to not more than about 80%.

The agent of the present invention can be used effectively as a prophylactic or therapeutic agent of diabetes (e.g., type 1 diabetes, type 2 diabetes, gestational diabetes etc.), hyperlipidemia (e.g., hypertriglyceridemia, hypercholesterolemia, hypo-high-density-lipoproteinemia, postprandial hyperlipidemia etc.), diabetic complications (e.g., neuropathy, nephropathy, retinopathy, cataract, macroangiopathy, osteopenia etc.), impaired glucose tolerance (IGT), obesity, osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, hypertension, polycystic ovary syndrome, gestational diabetes, renal diseases (e.g., diabetic nephropathy, glomerular nephritis, glomerulosclerosis, nephrotic syndrome, hypertensive nephrosclerosis, terminal renal diabetes etc.), muscular dystrophy, cardiac infarction, angina, cerebrovascular disease (e.g., cerebral infarction, stroke), insulin resistance syndrome, syndrome X, hyperinsulinemia, sensory disorders in hyperinsulinemia, tumor (e.g., leukemia, breast cancer, prostate cancer, cutaneous cancer etc.), irritable bowel syndrome, acute or chronic diarrhea, visceral fat syndrome and the like. In addition, the agent of the present invention can be also used for treatment aiming at improving insulin resistance, enhancing insulin sensitivity, or preventing progress of impaired glucose tolerance into diabetes. Furthermore, the agent of the present invention can be also used for controlling appetite and food intake in the patients under diabetic treatments.

For diagnostic criteria of diabetes, Japan Diabetes Society reported new diagnostic criteria in 1999.

According to this report, diabetes is a condition showing any of a fasting blood glucose level (glucose concentration in venous plasma) of not less than 126 mg/dl, a 75 g oral glucose tolerance test (75 g OGTT) 2 h level (glucose concentration in venous plasma) of not less than 200 mg/dl, a non-fasting blood glucose level (glucose concentration in venous plasma) of not less than 200 mg/dl. A condition not falling under the above-mentioned diabetes, and not being "a condition showing a fasting blood glucose level (glucose concentration in venous plasma) of less than 110 mg/dl or a 75 g oral glucose tolerance test (75 g OGTT) 2 h level (glucose concentration in venous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing a fasting blood glucose level (glucose concentration in venous plasma) of not less than 126 mg/dl and a 75 g oral glucose tolerance test 2 h level (glucose concentration in venous plasma) of not less than 200 mg/dl.

According to the above-mentioned reports, impaired glucose tolerance is a condition showing a fasting blood glucose level (glucose concentration in venous plasma) of less than 126 mg/dl and a 75 g oral glucose tolerance test 2 h level (glucose concentration in venous plasma) of not less than 140 mg/dl and less than 200 mg/dl. According to the report of ADA, a condition showing a fasting blood glucose level (glucose concentration in venous plasma) of not less than 110 mg/dl and less than 126 mg/dl is called IFG (Impaired Fasting Glucose). According to the report of WHO, among the IFG (Impaired Fasting Glucose), a condition showing a 75g oral glucose tolerance test 2 h level (glucose concentration in venous plasma) of less than 140 mg/dl is called IFG (Impaired Fasting Glycemia).

The agent of the present invention can be also used as a prophylactic and therapeutic agent of diabetes, borderline type, impaired glucose tolerance, IFG (Impaired Fasting Glucose) and IFG (Impaired Fasting Glycemia), as determined according to the above-mentioned new diagnostic criteria. Moreover, the agent of the present invention can prevent progress of borderline type, impaired glucose tolerance, IFG (Impaired Fasting Glucose) or IFG (Impaired Fasting Glycemia) into diabetes.

Moreover, the agent of the present invention can be used in combination with a pharmaceutical agent (hereinafter abbreviated as combination agent) such as a therapeutic agent of diabetes, a therapeutic agent of diabetic complications, an anti-hyperlipidemia agent, an antihypertensive agent, a diuretic, a chemotherapeutant, an immunotherapeutic agent and the like. The agent of the present invention itself can contain such combination agent. Unless particularly specified in the present specification, by the mere "combination" is meant both a mode of administration using separate pharmaceutical agents and a mode of a single, combined pharmaceutical agent. When separate pharmaceutical agents are combined for use, the agent of the present invention and a combination agent are not limited as to the administration time. These may be simultaneously administered to a subject of administration or may be administered at staggered times. Two or more kinds of the combination agents may be combined at an appropriate ratio for use.

The dose of a combination agent can be determined as appropriate with the dose clinically employed for each pharmaceutical agent as a standard. The mixing ratio of the agent of the present invention and a combination agent can be determined appropriately depending on the subject of administration, administration route, target disease, condition, combination and the like.

Examples of the therapeutic agent of diabetes include insulin preparations (e.g., animal insulin preparation extracted from pancreas of cow or pig; human insulin preparation synthesized by genetic engineering techniques using *Escherichia coli* or yeast etc.), α-glucosidase inhibitor (e.g., voglibose, acarbose, miglitol, emiglitate etc.), biguanide agent (e.g., phenformin, metformin, buformin etc.), insulin secretagogue [e.g., sulfonylurea agent (e.g., tolbutamide, glibenclamide, gliclazide, chlorpropamide, tolazamide, acetohexamide, glyclopyramide, glimepiride, glipizide, glybuzole etc.), repaglinide, senaglinide, nateglinide, mitiglinide or calcium salt hydrate thereof, GLP-1 and the like], amylin agonist (e.g., pramlintide etc.), phosphotyrosine phosphatase inhibitor (e.g., vanadic acid etc.) and the like.

Examples of the therapeutic agent of diabetic complications include aldose reductase inhibitors (e.g., tolrestat, epalrestat, zenarestat, zopolrestat, minalrestat, fidarestat, SNK-860, CT-112 etc.), neurotrophic factors (e.g., NGF, NT-3, BDNF etc.), neurotrophic factor production promoter, PKC inhibitors (e.g., LY-333531 etc.), AGE inhibitors (e.g., ALT946, pimagedine, pyratoxathine, N-phenacylthiazolium bromide (ALT766), EXO-226 etc.), active oxygen scavengers (e.g., thioctic acid etc.) and cerebral vasodilators (e.g., tiapride, mexiletine etc.).

Examples of the anti-hyperlipidemia agent include statin compounds (e.g., pravastatin, simvatatin, lovastatin, atorvastatin, fluvastatin, cerivastatin, itavastatin, salts thereof (e.g., sodium salt) etc.), which are inhibitors of cholesterol synthesis, squalene synthetase inhibitor, fibrate compound having a triglyceride lowering effect (e.g., bezafibrate, clofibrate, simfibrate, clinofibrate etc.) and the like.

Examples of the antihypertensive agent include angiotensin converting enzyme inhibitors (e.g., captoril, enalapril, delapril etc.), angiotensin II antagonists (e.g., losartan, candesartan cilexetil, eprosartan, valsartan, termisartan, irbesartan, tasosartan etc.), calcium antagonists (e.g., manidipine, nifedipine, amlonidipine, efonidipine, nicardipine etc.) and the like.

Examples of the anti-obesity agent include central acting anti-obesity agents (e.g., dexfenfluramine, fenfluramine, fentermine, sibutramine, amfepramone, dexamphetamine, mazindol, phenylpropanolamine, clobenzorex etc.), pancreatic lipase inhibitor (e.g., orlistat etc.), β3 agonist (e.g., CL-316243, SR-58611-A, UL-TG-307, SB-226552, AJ-9677, BMS-196085, AZ40140 etc.), anorectic peptides (e.g., leptin, CNTF (ciliary neurotropic factor) etc.), cholecystikinin agonists (e.g., lintitript, FPL-15849 etc.) and the like.

Examples of the diuretic include xanthine derivatives (e.g., sodium salicylate and theobromine, calcium salicylate and theobromine etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide etc.), anti-aldosterone preparations (e.g., spironolactone, triamterene etc.), carbonate dehydratase inhibitors (e.g., acetazolamide etc.), chlorobenzenesulfonamide preparations (e.g., chlortalidone, mefruside, indapamide etc.), azosemide, isosorbide, ethacrynic acid, piretanide, bumetanide, furosemide and the like.

Examples of the chemotherapeutant include alkylation agents (e.g., cyclophosphamide, ifosphamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etopoxide and the like. Of these, furtulon, neofurtulon and the like, which are 5-fluorouracil derivatives, are preferable.

Examples of the immunotherapeutic agent include microorganism or bacterial components (e.g., muramyl dipeptide derivative, picibanil etc.), polysaccharides having immunostimulant activity (e.g., lenthinan, schizophyllan, krestin etc.), cytokines obtained by genetic engineering techniques (e.g., interferon, interleukin (IL) etc.), colony stimulating factors (e.g., granulocyte-colony stimulating factor, erythropoietin etc.) and the like. Of these, IL-1, IL-2, IL-12 and the like are preferable.

Moreover, pharmaceutical agents having a cachexia improving effect acknowledged in animal models and clinical situations, which include cyclooxygenase inhibitors (e.g., indomethacin etc.) [Cancer Research, vol. 49, pp. 5935-5939, 1989], progesterone derivatives (e.g., megestrol acetate) [Journal of Clinical Oncology, vol. 12, pp. 213-225, 1994], glucosteroid (e.g., dexamethasone etc.), metoclopramide agents, tetrahydrocannabinol agents (publications are the same as the above), fat metabolism improving agents (e.g., eicosapentanoic acid etc.) [British Journal of Cancer, vol. 68, pp. 314-318, 1993], growth hormone, IGF-1, and antibodies against TNF-α, LIF, IL-6 and oncostatin M, which induce cachexia, and the like, can be also used in combination with the pharmaceutical agent of the present invention.

Preferable examples of the combination agent include the following.
1) insulin preparation;
2) biguanide agent;
3) insulin secretagogue such as sulfonylurea agent and the like;
4) biguanide agent;
5) α-glucosidase inhibitor;
6) insulin preparation and biguanide agent;
7) insulin preparation and α-glucosidase inhibitor;
8) insulin secretagogue such as sulfonylurea agent and the like, and biguanide agent;
9) insulin secretagogue such as sulfonylurea agent and the like, and α-glucosidase inhibitor;
10) biguanide agent and α-glucosidase inhibitor;
11) insulin sensitizers (e.g., PPARγ agonist like substance);
12) combinations of an insulin sensitizer and the agent of the above-mentioned 1)-10);
13) hypoglycemic agent and other therapeutic agents of diabetic complications;
14) the aforementioned other agents and combination of two or more kinds thereof.

When the agent of the present invention is used in combination with a combination agent, the dose thereof can be reduced within a safe range in consideration of the adverse effect of these agents. Particularly, the dose of insulin preparation, insulin secretagogue such as sulfonylurea agent and the like, and biguanide agent can be reduced to a lower level as compared to the general dose thereof. Consequently, the side effects possibly caused by these agents can be prevented safely. In addition, the dose of the agents for diabetic complications, anti-hyperlipidemia agents and antihypertensive agents can be reduced, as a result of which the side effects possibly caused by these agents can be prevented effectively.

### Best Mode for Carrying Out the Invention

The present invention is explained in more detail by the following Production Examples, Preparation Examples, Experimental Examples and Formulation Examples, which are not to be construed as limitative. In the following description, % means percent by weight and room temperature means 1°C to 30°C, unless otherwise specified.

When a base, an amino acid and the like are expressed using abbreviations in the present specification, they are based on the abbreviations of IUPAC-IUB Commission on Biochemical Nomenclature or conventional abbreviations used in the pertinent field, which are exemplified by the following. When an amino acid has an optical isomer, it refers to an L form, unless specifically indicated.

In the Sequence Listing in the present specification, SEQ ID Nos show the following sequences.
[SEQ ID No: 1] depicts the base sequence of primer PARD-U used in Preparation Example 1.
[SEQ ID No: 2] depicts the base sequence of primer PARD-L used in Preparation Example 1.
[SEQ ID No: 3] depicts the base sequence of primer XRA-U used in Preparation Example 2.
[SEQ ID No: 4] depicts the base sequence of primer XRA-L used in Preparation Example 2.
[SEQ ID No: 5] depicts the base sequence of PPRE-U used in Preparation Example 4.
[SEQ ID No: 6] depicts the base sequence of PPRE-L used in Preparation Example 4.
[SEQ ID No: 7] depicts the base sequence of primer TK-U used in Preparation Example 4.
[SEQ ID No: 8] depicts the base sequence of primer TK-L used in Preparation Example 4.
[SEQ ID No: 9] depicts the base sequence of primer PAG-U used in Preparation Example 6.
[SEQ ID No: 10] depicts the base sequence of primer PAG-L used in Preparation Example 6.

### Examples

### Production Example 1: Production of 4-(5-methyl-2-phenyl-4-oxazolylmethoxy)benzyl alcohol

To a solution of 4-(5-methyl-2-phenyl-4-oxazolylmethoxy)benzaldehyde (33.42 g) in methanol (150 ml) - tetrahydrofuran (30 ml) was slowly added sodium borohydride (4.31 g) at 0°C. After stirring the mixture at room temperature for 30 min, water was added to the reaction mixture and the mixture was stirred for 1 h. The crystals of the precipitated 4-(5-methyl-2-phenyl-4-oxazolylmethoxy)benzyl alcohol (32.85 g, yield 98%) were collected by filtration. Recrystallization from ethyl acetate-diethyl ether gave pale-yellow crystals, melting point: 128-129°C.

### Production Example 2: Production of 4-(4-chloromethylphenoxymethyl)-5-methyl-2-phenyloxazole

To a solution of 4-(5-methyl-2-phenyl-4-oxazolylmethoxy)benzyl alcohol (5.00 g) in toluene (40 ml) was added thionyl chloride (1.85 ml) and the mixture was stirred at room temperature for 30 min. Ice water was added to the reaction mixture and the mixture was extracted with ethyl acetate. The ethyl acetate layer was washed with water, dried over MgSO₄ and concentrated to give crystals (5.23 g, yield 99%) of 4-(4-chloromethylphenoxymethyl)-5-methyl-2-phenyloxazole. Recrystallization from ethyl acetate-hexane gave colorless crystals, melting point: 108-109°C.

### Production Example 3: Production of E-4-[4-(5-methyl-2-phenyl-4-oxazolylmethoxy)benzyloxyimino]-4-phenylbutyric acid

To a solution of methyl E-4-hydroxyimino-4-phenylbutyrate (661 mg) and 4-(4-chloromethylphenoxymethyl)-5-methyl-2-phenyloxazole (1.00 g) in N,N-dimethylformamide (10 ml) was added sodium hydride (60% in oil, 127 mg) at room temperature under a nitrogen atmosphere, and the mixture was stirred for 1 h. 1N Hydrochloric acid (5 ml) was added, aqueous sodium hydrogen carbonate was added and the mixture was extracted with ethyl acetate. The ethyl acetate layer was washed with saturated brine, dried over MgSO₄, and concentrated. The residue was subjected to silica gel column chromatography and an oily substance was obtained from the portion eluted with ethyl acetate-hexane (1:3, volume ratio). This was dissolved in tetrahydrofuran (10 ml) - methanol (5 ml), 1N aqueous sodium hydroxide solution (5 ml) was added and the mixture was stirred at room temperature for 1.5 h. 1N Hydrochloric acid (5.5 ml) was added to the reaction mixture and the mixture was extracted with ethyl acetate. The ethyl acetate layer was washed with saturated brine, dried over MgSO₄, and concentrated. The remaining crystals were recrystallized from ethyl acetate - hexane to give E-4-[4-(5-methyl-2-phenyl-4-oxazolylmethoxy)benzyloxyimino]-4-phenylbutyric acid to give colorless crystals (907 mg, yield 60%), melting point: 126-127°C (dec.).

### Preparation Example 1: Cloning of human PPARδ gene

Human PPARδ gene was cloned by PCR method using a primer set 
PARD-U; 5′-AAC GGT ACC TCA GCC ATG GAG CAG CCT CAG GAG G-
3′ (SEQ ID No: 1)
 
PARD-L; 5′-TAA GTC GAC CCG TTA GTA CAT GTC CTT GTA GAT C-
3′ (SEQ ID No: 2)
 prepared by referring to the base sequence of PPARδ gene reported by Schmidt, A. et al. (Mol Endocrinol 1992; 6: 1634-1641) and using pancreatic cDNA (Toyobo, QUICK-Clone cDNA) as a template.

The PCR reaction was carried out according to the Hot Start method using AmpliWax PCR Gem 100 (Takara Shuzo Co., Ltd.). As a lower layer mixture, 10×LA PCR Buffer (2 µl), 2.5 mM dNTP solution (3 µl), 12.5 µM primer solution (each 2.5 µl) and sterile distilled water (10 µl) were mixed. As an upper layer mixture, human heart cDNA (1 ng/ml, 1 µl) as a template, 10×LA PCR Buffer (3 µl), 2.5 mM dNTP solution (1 µl), TaKaRa LA Taq DNA polymerase (0.5 µl, Takara Shuzo Co., Ltd.) and sterile distilled water (24.5 µl) were mixed. To the prepared lower layer mixture was added one AmpliWax PCR Gem 100 (Takara Shuzo Co., Ltd.), and the mixture was treated at 70°C for 5 min and in ice for 5 min, after which the upper layer mixture was added to give a reaction mixture of PCR. A tube containing the reaction mixture was set in Thermal Cycler (Perkin Elmer) and treated at 95°C for 2 min. The cycle of 95°C for 15 sec and 68°C for 2 min was repeated 45 times and the reaction mixture was treated at 72°C for 8 min. The obtained PCR product was subjected to agarose gel (1%) electrophoresis, and a 1.4 kb DNA fragment containing PPARδ gene was recovered from the gel and inserted into pT7Blue-T vector (Takara Shuzo Co., Ltd.) to give a plasmid pTBT-hPPARδ.

### Preparation Example 2: Cloning of human RXRα gene cloning of human RXRα gene

Human RXRα gene was cloned by PCR method using a primer set 
XRA-U: 5'-TTA GAA TTC GAC ATG GAC ACC AAA CAT TTC CTG-3'
(SEQ ID No: 3)
 
XRA-L: 5'-CCC CTC GAG CTA AGT CAT TTG GTG CGG CGC CTC-3'
(SEQ ID No: 4)
 prepared by referring to the base sequence of RXRα gene reported by Mangelsdorf, D. J. et al. [Nature, vol. 345 (6272), pp. 224-229 (1990)] using kidney cDNA (produced by Toyobo, trademark: QUICK-Clone cDNA) as a template.

The PCR reaction was carried out according to the Hot Start method using AmpliWax PCR Gem 100 (produced by Takara Shuzo Co., Ltd.). As a lower layer mixture, 10×LA PCR Buffer (2 µl), 2.5 mM dNTP solution (3 µl), 12.5 µM primer solution (each 2.5 µl) and sterile distilled water (10 µl) were mixed. As an upper layer mixture, human kidney cDNA (1 ng/ml, 1 µl) as a template, 10×LA PCR Buffer (3 µl), 2.5 mM dNTP solution (1 µl), TaKaRa LA Taq DNA polymerase (0.5 µl, produced by Takara Shuzo Co., Ltd.) and sterile distilled water (24.5 µl) were mixed.

To the aforementioned lower layer mixture was added one AmpliWax PCR Gem 100 (produced by Takara Shuzo Co., Ltd.), and the mixture was treated at 70°C for 5 min and in ice for 5 min, after which the upper layer mixture was added to give a reaction mixture of PCR. A tube containing the reaction mixture was set in Thermal Cycler (produced by Perkin Elmer, USA) and treated at 95°C for 2 min. The cycle of 95°C for 15 sec and 68°C for 2 min was repeated 35 times and the reaction mixture was treated at 72°C for 8 min.

The obtained PCR product was subjected to agarose gel (1%) electrophoresis, and a 1.4 kb DNA fragment containing RXRα gene was recovered from the gel and inserted into pT7Blue-T vector (produced by Takara Shuzo Co., Ltd.) to give a plasmid pTBT-hRXRα.

### Preparation Example 3: Preparation of expression plasmids for human PPARδ, RXRα

A 7.8 kb FspI-NotI fragment of plasmid pVgRXR (Invitrogen) and a 0.9 kb FspI-NotI fragment containing the RXRα gene of the plasmid pTBT-hRXRα described in Preparation Example 2 were ligated to give a plasmid pVgRXR2. The pVgRXR2 was cleaved with BstXI and blunted at the end by T4 DNA polymerase (Takara Shuzo Co., Ltd.) treatment and cleaved with KpnI to give a 6.5 kb DNA fragment. Separately, the plasmid pTBT-hPPARδ described in Preparation Example 1 was cleaved with SalI and blunted at the end by T4 DNA polymerase (Takara Shuzo Co., Ltd.) treatment and cleaved with KpnI to give a 1.4 kb DNA fragment containing PPARδ gene. By ligating the both DNA fragments, a plasmid pVgRXR2-hPPARδ was constructed.

### Preparation Example 4: Preparation of reporter plasmid Preparation of reporter plasmid

A DNA fragment containing a PPAR responsive element (PPRE) of acyl CoA oxidase was prepared using the following 5' terminal phosphorylated synthetic DNA

PPRE-U: 5′-pTCGACAGGGGACCAGGACAAAGGTCACGTTCGGGAG-3′ (SEQ
ID No: 5)

PPRE-L: 5'-pTCGACTCCCGAACGTGACCTTTGTCCTGGTCCCCTG-3' (SEQ
ID No: 6).

First, PPRE-U and PPRE-L were annealed and inserted into the SalI region of the plasmid pBlueScript SK+. The base sequence of the inserted fragment was determined, based on which plasmid pBSS-PPRE4, containing four PPREs ligated in tandem, was selected.

HSV Thymidine kinase minimum promoter (TK promoter) region was cloned by PCR method using pRL-TK vector (produced by Promega, USA) as a template and a primer set TK-U: 5'-CCCAGATCTCCCCAGCGTCTTGTCATTG-3' (SEQ ID No: 7) TK-L: 5'-TCACCATGGTCAAGCTTTTAAGCGGTC-3' (SEQ ID No: 8) prepared by referring to the base sequence of the promoter region of thymidine kinase gene reported by Luckow, B. et al. [Nucleic Acids Res., Vol. 15 (13), p. 5490 (1987)].

The PCR reaction was carried out according to the Hot Start method using AmpliWax PCR Gem 100 (Takara Shuzo Co., Ltd.). As a lower layer mixture, 10×LA PCR Buffer (2 µl), 2.5 mM dNTP solution (3 µl), 12.5 µM primer solution (each 2.5 µl) and sterile distilled water (10 µl) were mixed. As an upper layer mixture, pRL-TK vector (produced by Promega, USA, 1 µl) as a template, 10×LA PCR Buffer (3 µl), 2.5 mM dNTP solution (1 µl), TaKaRa LA Taq DNA polymerase (0.5 µl, produced by Takara Shuzo Co., Ltd.) and sterile distilled water (24.5 µl) were mixed.

To the prepared lower layer mixture was added one AmpliWax PCR Gem 100 (produced by Takara Shuzo Co., Ltd.), and the mixture was treated at 70°C for 5 min and in ice for 5 min, after which the upper layer mixture was added to give a reaction mixture of PCR. A tube containing the reaction mixture was set in Thermal Cycler (produced by Perkin Elmer, USA) and treated at 95°C for 2 min. The cycle of 95°C for 15 sec and 68°C for 2 min was repeated 35 times and the reaction mixture was treated at 72°C for 8 min.

The obtained PCR product was subjected to agarose gel (1%) electrophoresis, and a 140 b DNA fragment containing TK promoter was recovered from the gel and inserted into pT7Blue-T vector (produced by Takara Shuzo Co., Ltd.). A fragment containing the TK promoter, which was obtained by cleaving this plasmid with restriction enzymes BglII and NcoI, was ligated with a BglII-NcoI fragment of plasmid pGL3-Basic vector [produced by Promega, USA] to give a plasmid pGL3-TK.

The NheI-XhoI fragment (4.9 kb) of the obtained plasmid pGL3-TK and the NheI-XhoI fragment (200 b) of plasmid pBSS-PPRE4 were ligated to give a plasmid pGL3-4ERPP-TK.

This plasmid pGL3-4ERPP-TK was cleaved with BamHI (produced by Takara Shuzo Co., Ltd.) and blunted at the end by T4 DNA polymerase (produced by Takara Shuzo Co., Ltd.) treatment to give a DNA fragment.

The pGFP-C1 (produced by Toyobo) was cleaved with Bsu36I (NEB) and blunted at the end by T4 DNA polymerase (produced by Takara Shuzo Co., Ltd.) treatment to give a 1.6 kb DNA fragment.

By ligating the both DNA fragments, a reporter plasmid pGL3-4ERPP-TK neo was constructed.

### Preparation Example 5: Introduction of plasmids for expression of human PPARδ, RXRα and reporter plasmid into CHO-K1 cells and obtainment of expression cell

CHO-K1 cells were grown in a tissue culture flask (750 ml, Corning) using Ham's F12 Medium (Nissui Pharmaceutical Co., Ltd.) containing 10% fetal bovine serum (Lifetech Oriental), and stripped by treatment with 0.5 g/L tripsin-0.2 g/L EDTA (Lifetech Oriental). The cells were washed with PBS (Lifetech Oriental), centrifuged (1000 rpm, 5 min) and suspended in PBS. Using a gene pulser (Bio-Rad Laboratories), DNA was introduced into the cells under the following conditions. To be specific, 8 × 10⁶ cells, 10 µg of expression plasmid pVgRXR2-hPPARδ produced in Preparation Example 3 and 10 µg of reporter plasmid pGL3-4ERPP-TK neo produced in Preparation Example 4 were placed in a cuvette having a 0.4 cm gap and electropolated at 0.25 kV voltage and 960 mF capacitance. Thereafter, the cells were placed in the 10% fetal bovine serum-containing Ham's F12 Medium, cultured for 24 h, stripped again and centrifuged, suspended in Ham's F12 Medium containing 10% fetal bovine serum, which had been supplemented with geneticin (500 µg/ml, Lifetech Oriental) and zeocin (250 µg/ml, Invitrogen), diluted to 10⁴ cell/ml, inoculated to 96 well plate (Becton Dickinson) and cultured in a carbon dioxide gas incubator at 37°C to give a geneticin, zeocin resistant transformed strain.

The obtained transformed strain was cultured in a 24 well plate (corning). 10 mM Iloprost was added thereto and a strain, PPARδ:RXRα:4ERPP/CHO-K1, was selected, in which luciferase expression had been induced.

### Preparation Example 6: Cloning of human PPARγ gene

Human PPARγ gene was cloned by PCR method using a primer set 
PAG-U: 5′-GTG GGT ACC GAA ATG ACC ATG GTT GAC ACA GAG-3′
(SEQ ID No: 9)
 
PAG-L: 5′-GGG GTC GAC CAG GAC TCT CTG CTA GTA CAA GTC-3′
(SEQ ID No: 10)
 prepared by referring to the base sequence of PPARγ gene reported by Greene et al. [Gene Expr., vol. 4 (4-5), pp. 281-299 (1995)] and using heart cDNA (produced by Toyobo, trademark: QUICK-Clone cDNA) as a template.

The PCR reaction was carried out according to the Hot Start method using AmpliWax PCR Gem 100 (produced by Takara Shuzo Co., Ltd.). As a lower layer mixture, 10×LA PCR Buffer (2 µl), 2.5 mM dNTP solution (3 µl), 12.5 µM primer solution (each 2.5 µl) and sterile distilled water (10 µl) were mixed. As an upper layer mixture, human heart cDNA (1 ng/ml, 1 µl) as a template, 10×LA PCR Buffer (3 µl), 2.5 mM dNTP solution (1 µl), TaKaRa LA Taq DNA polymerase (0.5 µl, produced by Takara Shuzo Co., Ltd.) and sterile distilled water (24.5 µl) were mixed.

To the prepared lower layer mixture was added one AmpliWax PCR Gem 100 (produced by Takara Shuzo Co., Ltd.), and the mixture was treated at 70°C for 5 min and in ice for 5 min, after which the upper layer mixture was added to give a reaction mixture of PCR. A tube containing the reaction mixture was set in Thermal Cycler (produced by Perkin Elmer, USA) and treated at 95°C for 2 min. The cycle of 95°C for 15 sec and 68°C for 2 min was repeated 35 times and the reaction mixture was treated at 72°C for 8 min.

The obtained PCR product was subjected to agarose gel (1%) electrophoresis, and a 1.4 kb DNA fragment containing PPARγ gene was recovered from the gel and inserted into pT7Blue-T vector (produced by Takara Shuzo Co., Ltd.) to give a plasmid pTBT-hPPARγ.

### Preparation Example 7: Preparation of expression plasmids for human PPARγ, RXRα

A 7.8 kb FspI-NotI fragment of plasmid pVgRXR (produced by Invitrogen, USA) and a 0.9 kb FspI-NotI fragment containing the RXRα gene of the plasmid pTBT-hRXRα obtained in Preparation Example 2 were ligated to give a plasmid pVgRXR2. The pVgRXR2 was cleaved with BstXI and blunted at the end by T4 DNA polymerase (produced by Takara Shuzo Co., Ltd.) treatment and cleaved with KpnI to give a 6.5 kb DNA fragment.

Separately, the plasmid pTBT-hPPARγ obtained in Preparation Example 6 was cleaved with SalI and blunted at the end by T4 DNA polymerase (produced by Takara Shuzo Co., Ltd.) treatment and cleaved with KpnI to give a 1.4 kb DNA fragment containing human PPARγ gene.

By ligating the both DNA fragments, a plasmid pVgRXR2-hPPARγ was constructed.

### Preparation Example 8: Introduction of plasmids for expression of human PPARγ, RXRα and reporter plasmid into CHO-K1 cells and obtainment of expression cell

CHO-K1 cells were grown in a tissue culture flask (750 ml, produced by Corning Coaster Corporation, USA) using Ham's F12 Medium (produced by Nissui Pharmaceutical Co., Ltd.) containing 10% fetal bovine serum (produced by Life Technologies, Inc., USA), and stripped by treatment with 0.5 g/L tripsin-0.2 g/L EDTA (ethylenediamine tetraacetic acid, produced by Life Technologies, Inc., USA). The cells were washed with PBS, centrifuged (1000 rpm, 5 min) and suspended in PBS. Using a gene pulser (produced by Bio-Rad Laboratories, USA), DNA was introduced into the cells under the following conditions.

To be specific, 8 × 10⁶ cells, 10 µg of plasmid pVgRXR2-hPPARγ obtained in Preparation Example 7 and 10 µg of reporter plasmid pGL3-4ERPP-TK neo obtained in Preparation Example 4 were placed in a cuvette having a 0.4 cm gap and electropolated at 0.25 kV voltage and 960 µF capacitance. Thereafter, the cells were placed in the 10% fetal bovine serum-containing Ham's F12 Medium, cultured for 24 h, stripped again and centrifuged, suspended in Ham's F12 Medium containing 10% fetal bovine serum, which had been supplemented with geneticin (500 µg/ml, produced by Life Technologies, Inc. USA) and zeocin (250 µg/ml, produced by Invitrogen, USA), diluted to 10⁴ cell/ml, inoculated to 96 well plate (produced by Corning Costar Corporation, USA) and cultured in a carbon dioxide gas incubator at 37°C to give a geneticin, zeocin resistant transformant.

The obtained transformed strain was cultured in a 24 well plate (produced by Corning Costar Corporation, USA). 10 µM Pioglitazone hydrochloride was added thereto and a strain, PPARγ:RXRα:4ERPP/CHO-K1, was selected, in which luciferase expression had been induced.

### Experimental Example 1: PPARδ-RXRα heterodimer ligand activity of the compound of Production Example 3

The PPARδ:RXRα:4ERPP/CHO-K1 cells obtained in Preparation Example 5 were cultured in Ham's F12 medium (produced by Nissui Pharmaceutical Co., Ltd.) containing 10% fetal bovine serum [produced by Life Technologies, Inc., USA] and inoculated to a 96 well white plate [produced by Corning Coster Corporation, USA] at 2 × 10⁴ cells/well and cultured overnight at 37°C in a carbon dioxide gas incubator.

After washing the 96 well white plate with PBS (Phosphate-buffered saline), Ham's F12 medium containing 90 µl of 0.1% fatty acid-free bovine serum albumin (BSA) and a test compound (compound of Production Example 3, 10 µl) were added, and the plate was incubated at 37°C in a carbon dioxide gas incubator for 48 h. After removal of the medium, PicaGene7.5 (produced by Waco Pure Chemicals Industries, Ltd., 40 µl) was added, and after stirring, the luciferase activity was determined using Lumistar [produced by BMG Labtechnologies GmbH, Germany].

The induction rate was calculated from the luciferase activity of each test compound relative to the luciferase activity of the compound non-administration group as 1. The concentration of the test compound and induction rate were analyzed using PRISM 2.01 [produced by GraphPad Software, Inc., USA], based on which the EC₅₀ value (concentration of the compound at which induction rate shows 50% of the maximum value) of the test compound was calculated. As a result, the value was 4.4 µM.

### Experimental Example 2: PPARδ-RXRα heterodimer ligand activity of rosiglitazone

In the same manner as in the above-mentioned Experimental Example 1, the EC₅₀ value (concentration of the compound at which induction rate shows 50% of the maximum value) of rosiglitazone was calculated. As a result, the value was 146 µM.

### Experimental Example 3: PPARγ-RXRα heterodimer ligand activity of compound of Production Example 3

The PPARγ:RXRα:4ERPP/CHO-K1 cells obtained in Preparation Example 8 were cultured in Ham's F12 medium (produced by Nissui Pharmaceutical Co., Ltd.) containing 10% fetal bovine serum [produced by Life Technologies, Inc., USA] and inoculated to a 96 well white plate [produced by Corning Coster Corporation, USA] at 2 × 10⁴ cells/well and cultured overnight at 37°C in a carbon dioxide gas incubator.

After washing the 96 well white plate with PBS (Phosphate-buffered saline), Ham's F12 medium containing 90 µl of 0.1% fatty acid-free bovine serum albumin (BSA) and a test compound (compound of Production Example 3, 10 µl) were added, and the plate was incubated at 37°C in a carbon dioxide gas incubator for 48 h. After removal of the medium, PicaGene7.5 (produced by Waco Pure Chemicals Industries, Ltd., 40 µl) was added, and after stirring, the luciferase activity was determined using Lumistar [produced by BMG Labtechnologies GmbH, Germany].

The induction rate was calculated from the luciferase activity of each test compound relative to the luciferase activity of the compound non-administration group as 1. The concentration of the test compound and induction rate were analyzed using PRISM 2.01 [produced by GraphPad Software, Inc., USA], based on which the EC₅₀ value (concentration of the compound at which induction rate shows 50% of the maximum value) of the test compound was calculated. As a result, the value was 0.024 µM. The compound of Production Example 3 showed a superior PPARγ-RXRα heterodimer ligand activity.

### Experimental Example 4: PPARγ-RXRα heterodimer ligand activity of rosiglitazone

In the same manner as in the above-mentioned Experimental Example 3, the EC₅₀ value (concentration of the compound at which induction rate shows 50% of the maximum value) of rosiglitazone was calculated. As a result, the value was 0.028 µM.

### Experimental Example 5: Body weight gain-reducing action

The same blood-glucose-lowering dose of rosiglitazone or the compound of Production Example 3 was given to 11 or 12-week-old female KKAy mice in admixture with the feed for 4 days.

Rosiglitazone significantly increased the body weight at a dose that reduced the blood glucose level of the object mice to 50-58% (16.2 mg/kg body weight/day for rosiglitazone; 4.4 mg/kg body weight/day for compound of Production Example 3), but the compound of Production Example 3, confirmed to be the agonist of both PPARδ and PPARγ, did not show any significant body weight gain. The results are shown in Table 1.

**Table 1**

| | Initial body weight (g) | body weight (g) 4 days later | body weight gain (g/4 days) | % control | blood glucose level (mg/dl) |
|---|---|---|---|---|---|
| Control | 46.0±1.3 | 46.3±1.9 | 0.4±0.7 | 100 | 435±77 |
| rosiglitazone | 45.9±1.8 | 49.9±2.8 | 3.9±1.2* | 58 | 253±36 |
| Control | 48.4±2.1 | 49.5±2.1 | 1.1±0.7 | 100 | 540±79 |
| Compound of Production Example 3 | 48.9±1.8 | 51.5±2.6 | 2.1±1.1 | 50 | 272±55 |

| | | | | | |
|---|---|---|---|---|---|
| Figures in the Table: mean±standard deviation (4 or 5 in each group) *: significant difference from control (p < 0.05) | | | | | |

| **Formulation Example 1:** (production of capsule) | |
|---|---|
| 1) compound of Production Example 3 | 30 mg |
| 2) fine powder cellulose | 10 mg |
| 3) lactose | 19 mg |
| 4) magnesium stearate | 1 mg |
| | total 60 mg |

1), 2), 3) and 4) were mixed and filled into a gelatin capsule.

| **Formulation Example 2:** (production of tablet) | |
|---|---|
| 1) compound of Production Example 3 | 30 g |
| 2) lactose | 50 g |
| 3) corn starch | 15 g |
| 4) carboxymethylcellulose | 44 g |
| 5) magnesium stearate | 1 g |
| | 1000 tablets total 140 g |

The entire amount of 1), 2) and 3) and 30 g of 4) were kneaded with water, dried in vacuo and milled. The milled powder was admixed with 14 g of 4) and 1 g of 5) and the mixture was tableted by a tableting machine, whereby 1000 tablets each containing 30 mg of the compound of Production Example 3 were obtained.

### Industrial Applicability

In the treatment of diabetes and other diseases, administration of a medicine having a PPARγ agonist action and effective for the treatment and the like tends to increase body weight of the patients, though the target disease is progressively cured. However, the agent of the present invention can inhibit body weight gain of such patients. A pharmaceutical agent having both a PPARδ agonist-like action and a PPARγ agonist-like action can significantly inhibit body weight gain of patients, while treating diabetes and the like.

## Claims

1. An agent for inhibiting a body weight gain derived from a PPARγ agonist-like substance, which contains a PPARδ agonist-like substance.

2. The agent according to claim 1, wherein the PPARδ agonist-like substance and the PPARγ agonist-like substance are the same substance.

3. The agent according to claim 1, wherein the same substance is a compound represented by the formula 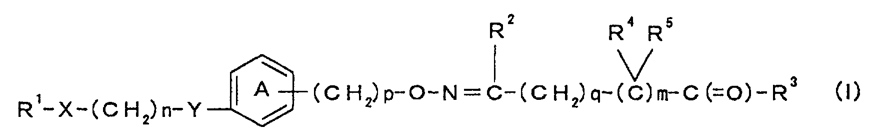 wherein
R¹ is an optionally substituted hydrocarbon group or an optionally substituted heterocyclic group;
X is a bond, a group of the formula: -CO-, -CH(OH)- or -NR⁶- (R⁶ is a hydrogen atom or an optionally substituted alkyl group) ;
n is an integer of 1 to 3;
Y is an oxygen atom, a sulfur atom, a group represented by -SO-, -SO₂- or -NR⁷- (R⁷ is a hydrogen atom or an optionally substituted alkyl group) ;
ring A is a benzene ring optionally having 1 to 3 additional substituent(s) ;
p is an integer of 1 to 8;
R² is a hydrogen atom, an optionally substituted hydrocarbon group or an optionally substituted heterocyclic group;
q is an integer of 0 to 6;
m is 0 or 1;
R³ is a hydroxy group, -OR⁸ (R⁸ is an optionally substituted hydrocarbon group) or -NR⁹R¹⁰ (R⁹ and R¹⁰ are the same or different and each is a hydrogen atom, an optionally substituted hydrocarbon group, an optionally substituted heterocyclic group, or an optionally substituted acyl group and R⁹ and R¹⁰ are optionally bonded to form a ring); and
R⁴ and R⁵ are the same or different and each is a hydrogen atom, an optionally substituted hydrocarbon group, and R⁴ and R² are optionally bonded to form a ring,
or a salt thereof.

4. The agent according to claim 1, wherein the same substance is E-4-[4-(5-methyl-2-phenyl-4-oxazolylmethoxy)benzyloxyimino]-4-phenylbutyric acid.

5. The agent according to claim 2, which is for the treatment of diabetes.

6. The agent according to claim 2, which is for the treatment of diabetic complications.

7. A method for inhibiting a body weight gain derived from a PPARγ agonist-like substance, which comprises administering an effective amount of a PPARδ agonist-like substance to a mammal.

8. Use of a PPARδ agonist-like substance for the production of an inhibitor of a body weight gain derived from a PPARγ agonist-like substance.

9. A therapeutic agent of diabetes comprising a PPARδ agonist-like substance and a PPARγ agonist-like substance, which inhibits a body weight gain derived from the PPARγ agonist-like substance to not more than about 80%.

10. The agent according to claim 9, wherein the PPARδ agonist-like substance and the PPARγ agonist-like substance are the same substance.
